# EUROPEAN PATENT SPECIFICATION

(11) **EP 1 878 805 B1**
(45) Date of publication and mention of the grant of the patent: **12.05.2010**
(21) Application number: 07007809.2
(22) Date of filing: 31.07.1998
(51) Int. Cl.: C12Q 1/70, C07K 7/00, C07K 14/00, C07K 14/16, G01N 33/569

(54) **Synthetic antigen for the detection of antibodies immunoreactive with HIV virus**
Synthetisches Antigen zum Nachweis von HIV-immunreaktiven Antikörpern
Antigène de synthèse pour la détection d'anticorps présentant une immunoréactivité vis-à-vis du virus VIH

(30) Priority: 01.08.1997 US 904826
(43) Date of publication of application: 16.01.2008
(62) Divisional of application: 98938310.4
(73) Proprietor: Bio-Rad Laboratories, Inc., Hercules, CA 94547 (US)
(72) Inventor: Coleman, Patrick F., Edmonds, Washington 98026 (US); Cole, Carol-Ann, Seattle, Washington 98112 (US); Chong-Dug Su, Peter, Seattle, Washington 98115 (US); Goshorn, Alice, Kamp, Seattle, Washington 98177 (US); Monji, Nobuo, Seattle, Washington 98115 (US)
(74) Representative: Gowshall, Jonathan Vallance

(56) References cited:
- WO-A-88/05440
- WO-A-94/20127
- RU-C- 2 043 411
- US-A- 5 075 211
- US-A- 5 580 739
- CLAVEL F ET AL: "Molecular cloning and polymorphism of the human immune deficiency virus type 2." NATURE. ENGLAND 1986 DEC 18-31, vol. 324, no. 6098, 18 December 1986 (1986-12-18), pages 691-695, XP000605290 ISSN: 0028-0836
- DATABASE BIOSIS [Online] BIOSCIENCES INFORMATION SERVICE, PHILADELPHIA, PA, US; 1995, MONJI NOBUO ET AL: "Specific and sensitive HIV-1/HIV-2 EIA using env and pol synthetic peptides." XP001105443 Database accession no. PREV199598347548 & CLINICAL CHEMISTRY, vol. 41, no. S6 PART 2, 1995, page S50, 47th Annual Meeting of the American Association for Clinical Chemistry, Inc.;Anaheim, California, USA; July 16-20, 1995 ISSN: 0009-9147
- POLLET D E ET AL: "CONFIRMATION AND DIFFERENTIATION OF ANTIBODIES TO HUMAN IMMUNODEFICIENCY VIRUS 1 AND 2 WITH A STRIP-BASED ASSAY INCLUDING RECOMBINANT ANTIGENS AND SYNTHETIC PEPTIDES" CLINICAL CHEMISTRY, vol. 37, no. 10 PART 1, 1991, pages 1700-1707, XP001105753 ISSN: 0009-9147
- WAIN-HOBSON S ET AL: "NUCLEOTIDE SEQUENCE OF THE AIDS VIRUS, LAV" CELL, MIT PRESS, CAMBRIDGE, MA,, US, vol. 40, no. 1, 1985, pages 9-17, XP000608742 ISSN: 0092-8674
- STERNBERG M J ET AL: "Prediction of antigenic determinants and secondary structures of the major AIDS virus proteins." FEBS LETTERS. NETHERLANDS 29 JUN 1987, vol. 218, no. 2, 29 June 1987 (1987-06-29), pages 231-237, XP001105722 ISSN: 0014-5793
- SPRENGERS E D ET AL: "A MICRO-ELISA SYSTEM FOR THE DETECTION OF BOTH ANTI-HIV-1 AND ANTI-HIV-2 ANTIBODIES" JOURNAL OF IMMUNOLOGICAL METHODS, vol. 139, no. 1, 1991, pages 77-82, XP001105718 ISSN: 0022-1759
- DATABASE BIOSIS [Online] BIOSCIENCES INFORMATION SERVICE, PHILADELPHIA, PA, US; 1992, IVANOV V S ET AL: "STUDY OF THE ANTIGENIC STRUCTURE OF HUMAN IMMUNODEFICIENCY VIRUSES BY MEANS OF SYNTHETIC PEPTIDES" XP002213768 Database accession no. PREV199294121790 & BIOORGANICHESKAYA KHIMIYA, vol. 18, no. 6, 1992, pages 784-793, ISSN: 0132-3423
- DATABASE BIOSIS [Online] BIOSCIENCES INFORMATION SERVICE, PHILADELPHIA, PA, US; 1995, MONJI N ET AL: "Sensitive and specific HIV-1/HIV-2 EIA using env and pol synthetic peptides." XP001105703 Database accession no. PREV199698616393 & TRANSFUSION (BETHESDA), vol. 35, no. 10 SUPPL., 1995, page 28S, 48th Annual Meeting of the American Association of Blood Banks;New Orleans, Louisiana, USA; November 11-15, 1995 ISSN: 0041-1132
- DATABASE SWISS-PROT [Online] XP002239590 Database accession no. P04584
- MYERS R A ET AL: "IDENTIFYING HIV-2-SEROPOSITIVE INDIVIDUALS BY REEVALUATING HIV-1 INDETERMINATE SERA" JOURNAL OF ACQUIRED IMMUNE DEFICIENCY SYNDROMES, RAVEN PRESS, NEW YORK, NY, US, vol. 5, no. 4, April 1992 (1992-04), pages 417-423, XP001105714 ISSN: 0894-9255
- MODROW S ET AL: "CARRIER BOUND SYNTHETIC OLIGOPEPTIDES IN ELISA TEST SYSTEMS FOR DISTINCTION BETWEEN HIV-1 AND HIV-2 INFECTION" JOURNAL OF ACQUIRED IMMUNE DEFICIENCY SYNDROMES, RAVEN PRESS, NEW YORK, US, vol. 2, no. 2, 1 April 1989 (1989-04-01), pages 141-148, XP000578021
- HICKMAN R K ET AL: "DETECTION AND DIFFERENTIATION OF HIV-1 GROUP O SERA FROM HIV-1 GROUP M AND HIV-2 USING RECOMBINANT ANTIGENS AND PEPTIDES" JOURNAL OF VIROLOGICAL METHODS, AMSTERDAM, NL, vol. 72, no. 1, May 1998 (1998-05), pages 43-49, XP000866196 ISSN: 0166-0934

## Description

### Field of the Invention

This invention relates to synthetic polypeptides, recombinant polypeptides and recombinant polynucleotide sequences encoding the same, useful for detecting antibodies associated with human immunodeficiency virus type 1 (HIV-1) and/or type 2 (HIV-2) (as used herein, "HIV" used without reference to the type shall mean either or both types), and particularly relates to synthetic polypeptides which mimic antigenic epitopes of the gene products of the HIV polymerase region.

### Background of the Invention

Human immunodeficiency virus type 1 and 2 (HIV-1 and HIV-2) are known to cause acquired immune deficiency syndrome (AIDS). Both viruses apparently exhibit similar modes of transmission. HIV-1 and HIV-2 were both isolated in the early 1980's from African AIDS patients. Since then, cases have been found in most countries of the world. Because the HIV viruses exhibit rapid genetic drift, widely divergent strains are emerging. Thus, detection and treatment of variant strains has proven to be challenging and difficult.

Individuals with antibodies reactive with HIV-1 and/or HIV-2 are determined by immunoassays of the conventional sandwich ELISA format. These assays are comprised of an immobilized viral antigen, that may be comprised of viral lysate, retrovirus proteins or natural or synthetic polypeptides, that is contacted with blood or serum components suspected of containing HIV antibodies. While the existing commercial tests appear to have significantly diminished the transmission of HIV virus in blood products, each test configuration may have some disadvantages.

The possible disadvantages of viral lysate tests include: the need to grow and handle large quantities of live infectious virus; the possibility that the live virus might be incorporated into test materials; the variable nature of the resulting viral lysate; and the substantial number of false positive and false negative results that require additional confirmatory testing. These disadvantages may also be associated with the use of isolated viral proteins as antigens.

The use of synthetic polypeptides, (as used herein, "synthetic polypeptide" and "polypeptide" shall mean any one, all or a combination of the following: synthetic polypeptide, recombinant polypeptide, or polypeptide) which can be engineered to immunologically mimic antigenic epitopes of the HIV viruses, may avoid some of the above-mentioned disadvantages. One area of concern with the use of synthetic polypeptides (less than or equal to 60 residues in length) in diagnostic assays is the consideration that viral antigenic drift could result in the failure to detect HIV-1 or HIV-2 infected sera using these assays, presumably due to the limited presentation of viral epitopes. One method of guarding against such an occurrence is to include polypeptides from different immunodominant regions of the viral genome. Thus, synthetic polypeptides that immunologically mimic immunodominant regions of the HIV-1 and HIV-2 pol gene products are important additions to the already described polypeptides that mimic the env, gag, and pol proteins of HIV-1 and HIV-2. U.S. Patent Nos. 4,629,783 and 5,075,211 describe synthetic polypeptides that mimic antigenic determinants of HIV-1. Cosand U.S. Patent 5,075,211 describes synthetic polypeptides that immunologically mimic antigenic epitopes of HIV-1 proteins from the pol region, including two polypeptides which are similar to the polypeptides of this invention. In blood screening assays, the greater the immunoreactivity of the antigens used in the assay method, the less likely antibodies to a new variant or subtype of HIV-1 or HIV-2, present in a patient's sample, will be left undetected.

U.S. Patent 5,306,466 describes an "HIV-3 retrovirus" which was initially believed to be separate and distinct from HIV-1 and HIV-2. Researchers have since determined that the HIV-3 retrovirus is merely a particular subtype of HIV-1, now referred to as subtype O, or Group O (R. De Leys, et. al., J.Virol.: 1207-1216 (1990); L.G Gürtler, et. al., J.Virol.:1581-1585 (1994)).

By comparing various HIV-1 isolates researchers have shown that some regions of the genome are highly variable while others are reasonably well conserved. Similar polymorphisms have also been observed for HIV-2.

Despite the apparent similarities in disease state and transmission of HIV-1 and HIV-2 viruses, the virus types have been differentiated based on their genetic divergence. Based on genetic analysis viral isolates can be grouped according to their genetic homology to previous isolates. Today, HIV-1 and HIV-2 form the two main branches of the HIV genetic tree. DNA hybridization studies suggest that, while regions of extensive homology exist between HIV-1 and HIV-2, other regions seem very divergent. (Clavel et al, Science 233: 343 (1986)). In fact, HIV-2 has been shown to have, overall, only about 40% homology with HIV-1, and studies have shown little immunological cross reactivity between the envelope glycoproteins. The limited serologic cross reactivity between these viruses makes screening assays based on HIV-1 antigens insufficient for screening or diagnosis of HIV-2 infection in human sera.

### SUMMARY OF THE INVENTION

The invention is defined by the appended claims. Polypeptide sequences named ROD 124E1, ROD 124C2X, ROD C1X, RODC3X and ROD 124C5X capable of mimicking immunodominant regions of HIV-2 proteins, encoded in the polymerase region, have been identified. These polypeptides are useful in the preparation of reagents for the screening of blood and blood products for exposure to HIV viruses. The polypeptides can be used in various specific binding assays for the detection of antibodies to HIV-2 virus.

### BRIEF DESCRIPTION OF THE DRAWINGS

FIG. 1 depicts a plasmid map of one of the recombinant constructs of the present invention, more specifically that of pGEX/*pol*23.
FIG. 2 depicts a plasmid map of one of the recombinant constructs of the present invention, more specifically that of pGEX/*pol*7.
FIG. 3 depicts a plasmid map of one of the recombinant constructs of the present invention, more specifically that of pQE/*pol*23.
FIG. 4 depicts a plasmid map of one of the recombinant constructs of the present invention, more specifically that of pQE/*pol*7.
FIG. 5 depicts a plasmid map of one of the recombinant constructs of the present invention, more specifically that of pThioHis/*pol*23.
FIG. 6 depicts a plasmid map of one of the recombinant constructs of the present invention, more specifically that of pThioHis/*pol*7.

### DETAILED DESCRIPTION OF THE SPECIFIC EMBODIMENTS

Novel polypeptides are provided that immunologically mimic proteins encoded by the HIV-2 retroviruses.

In general, the term "polypeptide" or "peptide" used herein shall mean a chain of amino acid molecules possessing biological activity. The terms do not relate to a product of any specific length.

In addition, one or more amino acids may be added to the termini of an oligopeptide or polypeptide to provide for coupling to a support or a carrier.

Cysteine is particularly preferred to facilitate covalent coupling to other polypeptides or, for example, to form dimers by oxidation. To form polymers, it is preferred to have at least two cysteine residues present in the molecules being linked, preferably by utilizing cysteine residues added to the terminal portions of the polypeptides. Combinations of cysteine with intervening amino acid spacers are also useful. For example, two cysteine residues can be separated by one or more amino acid residue. Glycine residues are particularly useful and from one to three glycine residues may be employed between amino acids. Lysine residues have also been found to be useful as linkers and from one to three lysine residues may be used to couple the polypeptides to a solid phase alone or in combination with other amino acids.

In addition, the subject polypeptide sequences may differ from the natural sequence after being modified by terminal-NH₂ acylation, e.g. acetylation, or thioglycolic acid amidation, terminal-carboxy amidation, e.g. with ammonia or methylamine, to provide stability, increased hydrophobicity for linking or binding to a support or other molecule, or for polymerization.

Polypeptides derived from the polymerase region of HIV-1 are described below. The family of HIV-1 polypeptides are encoded by the genomic polynucleotide sequences (LAI or BRU isolate) encompassing base pairs (bp) 4448 through (bp) 4585 (numbering of Genbank HIVBRUCG; Accession K02013, BRU isolate) or is encoded in the pol open reading frame from about amino acid residue numbers 940 to about 985. Polypeptide I has the following polypeptide sequence: 
(I) BRU124E

W-X-Lys-Ile-Gln-Asn-Phe-Arg-Val-Tyr-Tyr-Arg-Asp-
Ser-Arg-Asp-Pro-Leu-Trp-Lys-Gly-Pro-Ala-Lys-
Leu-Leu-Trp-Lys-Gly-Glu-Gly-Ala-Y-Z
 wherein W is either a H of the amino terminal NH₂ group of the polypeptide or one or more additional amino acids bonded to the amino terminal NH₂ group of the polypeptide, the additional amino acids being selected to facilitate coupling of the polypeptide to a carrier protein or to a support; X is absent, Cys-Gly-Gly; or Lys-Lys, Y is absent, Cys or one or more amino acids added to facilitate coupling; and Z is OH or NH₂.

Polypeptide II, also designated BRU124EX, has the following polypeptide sequence: 
(II) BRU124EX

W-X-Leu-Gln-Lys-Gln-Ile-Thr-Lys-Ile-Gln-Asn-Phe-Arg-
Val-Tyr-Tyr-Arg-Asp-Ser-Arg-Asp-Pro-Leu-Trp-Lys-Gly-
Pro-Ala-Lys-Leu-Leu-Trp-Lys-Gly-Glu-Gly-Ala-Y-Z
 wherein W is either a H of the amino terminal NH₂ group of the polypeptide or one or more additional amino acids bonded to the amino terminal NH₂ group of the polypeptide, the additional amino acids being selected to facilitate coupling of the polypeptide to a carrier protein or to a support; X is absent, Cys-Gly-Gly; or Lys-Lys, Y is absent, Cys or one or more amino acids added to facilitate coupling; and Z is OH or NH₂.

Polypeptide III, also designated BRU124F1X, has the following polypeptide sequence: 
(III) BRU124F1X
W-X-Lys-Ile-Gln-Asn-Phe-Arg-Val-Tyr-Tyr-Arg-Asp-Ser-
Arg-Asp-Pro-Leu-Trp-Lys-Gly-Pro-Ala-Lys-Leu-Leu-Trp-
Lys-Gly-Glu-Gly-Ala-Val-Val-Ile-Gln-Asp-Asn-Ser-Asp-
Ile-Lys-Y-Z
 wherein W is either a H of the amino terminal NH₂ group of the polypeptide or one or more additional amino acids bonded to the amino terminal NH₂ group of the polypeptide, the additional amino acids being selected to facilitate coupling of the polypeptide to a carrier protein or to a support; X is absent, Cys-Gly-Gly; or Lys-Lys, Y is absent, Cys or one or more amino acids added to facilitate coupling; and Z is OH or NH₂.

Polypeptide IV, also designated BRU124F3X, has the following polypeptide sequence: 
(IV) BRU124F3X
W-X-Lys-Ile-Gln-Asn-Phe-Arg-Val-Tyr-Tyr-Arg-Asp-
Ser-Arg-Asp-Pro-Leu-Trp-Lys-Gly-Pro-Ala-Lys-
Leu-Leu-Trp-Lys-Gly-Glu-Gly-Ala-Val-Val-Ile-
Gln-Asp-Asn-Y-Z
 wherein W is either a H of the amino terminal NH₂ group of the polypeptide or one or more additional amino acids bonded to the amino terminal NH₂ group of the polypeptide, the additional amino acids being selected to facilitate coupling of the polypeptide to a carrier protein or to a support; X is absent, Cys-Gly-Gly; or Lys-Lys, Y is absent, Cys or one or more amino acids added to facilitate coupling; and Z is OH or NH₂.

The polypeptides of the invention, derived from the polymerase region of HIV-2 are described below. Polypeptide V, also designated ROD124E1, is encoded by the polynucleotide sequence of the HIV-2 genome encompassing base pairs (bp) 4694 through (bp) 4861 (numbering by Genbank HIV2ROD; Accession M15390, HIV-2ROD isolate) or is encoded in the pol open reading frame from about amino acid residue numbers 956 through 1001.

Polypeptide V has the following polypeptide sequence: 
(V) ROD124E1
W-X-Lys-Leu-Lys-Asp-Phe-Arg-Val-
Tyr-Phe-Arg-Glu-Gly-Arg-Asp-Gln-Leu-Trp-
 
Lys-Gly-Pro-Gly-Glu-Leu-Leu-Trp-Lys-Gly-
Glu-Gly-Ala-Y-Z
 wherein W is either a H of the amino terminal NH₂ group of the polypeptide or one or more additional amino acids bonded to the amino terminal NH₂ group of the polypeptide, the additional amino acids being selected to facilitate coupling of the polypeptide to a carrier protein or to a support; X is absent, Cys-Gly-Gly; or Lys-Lys, Y is absent, Cys or one or more amino acids added to facilitate coupling; and Z is OH or NH₂.

Polypeptide VI , also designated ROD124EX, has the following polypeptide sequence: 
(VI) ROD124EX

W-X-Leu-Gln-Ala-Lys-Asn-Ser-Lys-
Leu-Lys-Asp-Phe-Arg-Val-Tyr-Phe-Arg-Glu-
Gly-Arg-Asp-Gln-Leu-Trp-Lys-Gly-Pro-Gly-
Glu-Leu-Leu-Trp-Lys-Gly-Glu-Gly-Ala-Y-Z
 wherein W is either a H of the amino terminal NH₂ group of the polypeptide or one or more additional amino acids bonded to the amino terminal NH₂ group of the polypeptide, the additional amino acids being selected to facilitate coupling of the polypeptide to a carrier protein or to a support; X is absent, Cys-Gly-Gly; or Lys-Lys, Y is absent, Cys or one or more amino acids added to facilitate coupling; and Z is OH or NH₂.

Another polypeptide of the invention, Polypeptide VII , also designated ROD124C2X, has the following polypeptide sequence: 
(VII) ROD124C2X

W-X-Lys-Leu-Lys-Asp-Phe-Arg-Val-
Tyr-Phe-Arg-Glu-Gly-Arg-Asp-Gln-Leu-Trp-
Lys-Gly-Pro-Gly-Glu-Leu-Leu-Trp-Lys-Gly-
Glu-Gly-Ala-Val-Leu-Val-Lys-Val-Gly-Thr-
Asp-Ile-Lys-Y-Z
 wherein W is either a H of the amino terminal NH₂ group of the polypeptide or one or more additional amino acids bonded to the amino terminal NH₂ group of the polypeptide, the additional amino acids being selected to facilitate coupling of the polypeptide to a carrier protein or to a support; X is absent, Cys-Gly-Gly; or Lys-Lys, Y is absent, Cys or one or more amino acids added to facilitate coupling; and Z is OH or NH₂.

Another polypeptide of the invention, Polypeptide VIII , also designated ROD124C1X, has the following polypeptide sequence: 
(VIII) ROD124C1X
W-X-Tyr-Phe-Arg-Glu-Gly-Arg-Asp-Gln-Leu-Trp-Lys-Gly-Pro-Gly-
Glu-Leu-Leu-Trp-Lys-Gly-Glu-Gly-Ala-Val-Leu-Val-Lys-
Val-Gly-Thr-Asp-Ile-Lys-Y-Z
 wherein W is either a H of the amino terminal NH₂ group of the polypeptide or one or more additional amino acids bonded to the amino terminal NH₂ group of the polypeptide, the additional amino acids being selected to facilitate coupling of the polypeptide to a carrier protein or to a support; X is absent, Cys-Gly-Gly; or Lys-Lys, Y is absent, Cys or one or more amino acids added to facilitate coupling; and Z is OH or NH₂.

Another polypeptide of the invention, Polypeptide IX, also designated ROD123 C3X, has the following polypeptide sequence: 
(IX) ROD123C3X

X-Lys-Leu-Lys-Asp-Phe-Arg-Val-Tyr-Phe-Arg-
Glu-Gly-Arg-Asp-Gln-Leu-Trp-Lys-Gly-Pro-
Gly-Glu-Leu-Leu-Trp-Lys-Gly-Glu-Gly-Ala-
Val-Leu-Val-Lys-Val-Gly-Thr-Asp-Ile-Lys-Y-Z
 wherein X is either a H of the amino terminal NH₂ group of the polypeptide or one or more additional amino acids bonded to the amino terminal NH₂ group of the polypeptide, the additional amino acids being selected to facilitate coupling of the polypeptide to a carrier protein or to a support, Y is absent, Cys or one or more amino acids added to facilitate coupling; and Z is OH or NH₂.

Polypeptide X , also designated POL2A1, has the following polypeptide sequence: 
(X) POL2A1
W-X-Lys-Gly-Pro-Gly-Glu-Leu-Leu-
Trp-Lys-Gly-Glu-Gly-Ala-Val-Leu-Val-Lys-
Val-Gly-Thr-Asp-Ile-Lys-Ile-Ile-Pro-Arg-
Arg-Lys-Ala-Lys-Ile-Ile-Y-Z
 wherein W is either a H of the amino terminal NH₂ group of the polypeptide or one or more additional amino acids bonded to the amino terminal NH₂ group of the polypeptide, the additional amino acids being selected to facilitate coupling of the polypeptide to a carrier protein or to a support; X is absent, Cys-Gly-Gly; or Lys-Lys, Y is absent, Cys or one or more amino acids added to facilitate coupling; and Z is OH or NH₂.

Another polypeptide of the invention, Polypeptide XI , also designated ROD124C5X, has the following polypeptide sequence: 
(XI) ROD124C5X

W-X-Lys-Leu-Lys-Asp-Phe-Arg-Val-
Tyr-Phe-Arg-Glu-Gly-Arg-Asp-Gln-Leu-Trp-
Lys-Gly-Pro-Gly-Glu-Leu-Leu-Trp-Lys-Gly-
Glu-Gly-Ala-Val-Leu-Val-Lys-Val-Gly-Y-Z
 wherein W is either a H of the amino terminal NH₂ group of the polypeptide or one or more additional amino acids bonded to the amino terminal NH₂ group of the polypeptide, the additional amino acids being selected to facilitate coupling of the polypeptide to a carrier protein or to a support; X is absent, Cys-Gly-Gly; or Lys-Lys, Y is absent, Cys or one or more amino acids added to facilitate coupling; and Z is OH or NH₂.

Of particular interest is the use of the mercaptan group of cysteines or thioglycolic acids used for acylating terminal amino groups or the like for linking two of the polypeptides or oligopeptides or combinations thereof by a disulfide linkage or a longer linkage. To achieve this, compounds may be employed having bis-haloacetyl groups, nitroarylhalides, or the like where the reagents are specific for thio groups. Thus, the linking between the two mercapto groups of the different polypeptides or oligopeptides may be a single bond or a linking group of at least 2, usually at least 4, and not more than about 16, usually not more than about 14 carbon atoms.

Alternatively, the region of the viral genome coding for the polypeptide of the invention may be cloned by conventional recombinant DNA techniques and expressed. These techniques include PCR mediated cloning, as well as, synthesis of single strand polynucleotide strands encoding the polypeptide of interest. See generally, Maniatis et al, Molecular Cloning, A Laboratory Manual, CSH, Cold Spring Harbor Laboratory, 1982.

For expression, the coding sequence will be provided with start and stop codons, promoter and terminator regions and usually a replication system to provide an expression vector for expression in a cellular host, e.g., prokaryotic or eukaryotic, bacterial, yeast, mammal, etc.

The DNA sequence by itself, fragments thereof, or larger sequences, usually at least 15 bases, preferably at least 18 bases, may be used as probes for detection of retroviral RNA or proviral DNA. Numerous techniques are described, such as the Grunstein-Hogness technique, Southern technique, Northern technique, dot-blot, improvements thereon, as well as other methodology. See, for example, WO 83/02277 and Berent et al, Biotechniques (1985) 3:208.

Conveniently, the polypeptide may be prepared as a fused protein, where the polypeptide may be the N- or C- terminus of the fused polypeptide. Commonly, the polypeptide of interest is expressed as a "translational fusion," meaning that the expressed protein will have its amino terminus attached to a "partner" protein that is easily expressed in *E*. *coli,* or other well known expression systems. The fusion also may include a purification tag or "handle."

One example of such a fusion system is the use of a portion of the glutathione S-transferase gene which encodes a protein that can be used in affinity-purification on glutathione-agarose resin. This fusion system is found in the commercially available pGEX plasmid (Pharmacia). A second example is the use of a portion of the dihydrofolate reductase gene as the fusion partner and a sequence encoding six histidine residues as the handle. The "His-tag" allows the resulting fusion protein to be purified by Ni-NTA (nickel) chromatography. This fusion system is found in the commercially available pQE42 plasmid (QIAGEN). Similarly, a portion of the *E*. *coli* thioredoxin gene having a "His-patch" consisting of three histidine residues engineered into the thioredoxin coding sequence allows purification of expressed proteins by Ni-NTA chromatography. This fusion system is found in the commercially available pThioHisA plasmid (Invitrogen).

A resulting fused protein could be used directly by itself as the reagent, or the subject polypeptide may be cleaved from all or a portion of the remaining sequence of the fused protein. With a polypeptide containing no internal methionines, by introducing a methionine at the fusion site, the polypeptide may be cleaved employing cyanogen bromide. Where there is an internal methionine, it would be necessary to provide for a proteolytic cleavage site, e.g., polylysine and/or arginine or combinations thereof. Alternatively, the internal methionine could be substituted by an amino acid such as leucine and an N-terminal methionine added for cyanogen bromide cleavage. A wide variety of proteases, including dipeptidases, are well known, and the appropriate processing signal could be introduced at the proper site. The processing signal may have tandem repeats so as to insure cleavage, since the presence of one or more extraneous amino acids will not interfere with the utility of the subject polypeptides.

Recombinant polynucleotide sequences encoding a number of polypeptides, derived from the polymerase region of HIV-1 are described below. The sequences are derived from the HIV-1 genomic polynucleotide sequences (LAI isolate) encompassing base pairs (bp) 2549 through (bp) 3139, and base pairs 4391 (bp) through (bp) 4648, as found in the HIV-1 viral lysate.

Polynucleotide Sequence I, also know as *pol*23, has the following sequence:

(I) pol23

CCA GGG ATT AGA TAT CAG TAC AAT GTG CTT CCA CAG GGA TGG
AAA GGA TCA CCA GCA ATA TTC CAA AGT AGC ATG ACA AAA ATC
TTA GAG CCT TTT AGA AAA CAA AAT CCA GAC ATA GTT ATC TAT
CAA TAC ATG GAT GAT TTG TAT GTA GGA TCT GAC TTA GAA ATA
GGG CAG CAT AGA ACA AAA ATA GAG GAG CTG AGA CAA CAT CTG
TTG AGG TGG GGA CTT ACC ACA CCA GAC AAA AAA CAT CAG AAA
GAA CCT CCA TTC CTT TGG ATG GGT TAT GAA CTC CAT CCT GAT
AAA TGG ACA GTA CAG CCT ATA GTG CTG CCA GAA AAA GAC AGC
TGG ACT GTC AAT GAC ATA CAG AAG TTA GTG GGA AAA TTG AAT
TGG GCA AGT CAG ATT TAC CCA GGG ATT AAA GTA AGG CAA TTA
TGT AAA CTC CTT AGA GGA ACC AAA GCA CTA ACA GAA GTA ATA
CCA CTA ACA GAA GAA GCA GAG CTA GAA CTG GCA GAA AAC AGA
GAG ATT CTA AAA GAA CCA GTA CAT GGA GTG TAT TAT GAC CCA
TCA AAA GAC TTA ATA GCA GAA ATA CAG AAG CAG GGG CAA GGC
CAA

This sequence encodes the following amino acid sequence. (Without the intention of being bound by this teaching it is believed that the italicized amino acids represent particularly important residues to the immunoreactivity of the resulting polypeptide.)

(XII) pol23-aa Polypeptide Encoded by Polynucleotide Sequence (I) pol23

Pro-Gly-Ile-Arg-Tyr-Gln-Tyr-Asn-Val-Leu-Pro-Gln-Gly-Trp-Lys-Gly-Ser-Pro-Ala-Ile-
Phe-Gln-Ser-Ser-Met-Thr-Lys-Ile-Leu-Glu-Pro-Phe-Arg-Lys-Gln-Asn-Pro-Asp-Ile-Val
Ile-Tyr-Gln-Tyr-Met-Asp-Asp-Leu-Tyr-Val-Gly-Ser-Asp-Leu-Glu-Ile-Gly-Gln-His-Arg-
Thr-Lys-Ile-Glu-Glu-Leu-Arg-Gln-His-Leu-Leu-Arg-Trp-Gly-Leu-Thr-Thr-Pro-Asp-Lys-
Lys-His-Gln-Lys-Glu-Pro-Pro-Phe-Leu-Trp-Met-Gly-Tyr-Glu-Leu-His-Pro Asp-Lys-Trp-
Thr-Val-Gln-Pro-Ile-Val-Leu-Pro-Glu-Lys-Asp-Ser-Trp-Thr-Val-Asn-Asp-Ile-Gln-Lys-
Leu-Val-Gly-Lys-Leu-Asn-Trp-Ala-Ser-Gln-Ile-Tyr-Pro-Gly-Ile-Lys-Val-Arg-Gln-Leu-
Cys-Lys-Leu-Leu-Arg-Gly-Thr-Lys-Ala-Leu-Thr-Glu-Val-Ile-Pro-Leu-Thr-Glu-Glu-Ala-
Glu-Leu-Glu-Leu-Ala-Glu-Asn-Arg-Glu-Ile-Leu-Lys-Glu-Pro-Val-His-Gly-Val-Tyr-Tyr-
Asp-Pro-Ser-Lys-Asp-Leu-Ile-Ala-Glu-Ile-Gln-Lys-Gln-Gly-Gln-Gly-Gln

Another polynucleotide sequence of the invention, Polynucleotide Sequence II, also designated *pol*7*,* has the following sequence:

(II) pol7

TAC AGT GCA GGG GAA AGA ATA GTA GAC ATA ATA GCA ACA GAC
ATA CAA ACT AAA GAA TTA CAA AAA CAA ATT ACA AAA ATT CAA
AAT TTT CGG GTT TAT TAC AGG GAC AGC AGA GAT CCA CTT TGG
AAA GGA CCA GCA AAG CTC CTC TGG AAA GGT GAA GGG GCA GTA
GTA ATA CAA GAT AAT AGT GAC ATA AAA GTA GTG CCA AGA AGA
AAA GCA AAG ATC ATT AGG GAT TAT GGA AAA CAG ATG GCA GGT
GAT GAT

This sequence encodes the following amino acid sequence. (The italicized amino acids represent polypeptide BRU124F3X also disclosed herein.)

(XIII) pol7-aa Polypeptide Encoded by Polynucleotide Sequence (II) pol7

Tyr-Ser-Ala-Gly-Glu-Arg-Ile-Val-Asp-Ile-Ile-Ala-Thr-Asp-Ile-Gln-Thr-Lys-Glu-Leu-Gln-
Lys-Gln-Ile-Thr-Lys-Ile-Gln-Asn-Phe-Arg-Val-Tyr-Tyr-Arg-Asp-Ser-Arg-Asp-Pro-Leu-
Trp-Lys-Gly-Pro-Ala-Lys-Leu-Leu-Trp-Lys-Gly-Glu-Gly-Ala-Val-Val-Ile-Gln-Asp-Asn-
Ser-Asp-Ile-Lys-Val-Val-Pro-Arg-Arg-Lys-Ala-Lys-Ile-Ile-Arg-Asp-Tyr-Gly-Lys-Gln-
Met-Ala-Gly-Asp-Asp

The subject polypeptides may be employed linked to a soluble macromolecular (e.g., not less than 5kDal) carrier. Conveniently, the carrier may be a poly(amino acid), either naturally occurring or synthetic, to which antibodies are unlikely to be encountered in human serum. Illustrative polypeptides include poly-L-lysine, bovine serum albumin, keyhole limpet hemocyanin, bovine gamma globulin, etc. The choice is primarily one of convenience and availability.

With such conjugates, there will be at least one molecule of at least one subject polypeptide per macromolecule and not more than about 1 per 0.5 kDal, usually not more than about 1 per 2 kDal of the macromolecule. One or more different polypeptides may be linked to the same macromolecule.

The manner of linking is conventional, employing such reagents as p-maleimidobenzoic acid, p-methyldithiobenzoic acid, maleic acid anhydride, succinic acid anhydride, glutaraldehyde, etc. The linkage may occur at the N-terminus, C-terminus or at a site intermediate to the ends of the molecule. The subject polypeptide may be derivatized by linking, may be linked while bound to a support, or the like.

The polypeptides of the invention may be used as reagents in assays to detect antibodies to HIV-2 or antigens thereof. The polypeptides may be employed as labeled or unlabeled reagents depending upon their use (By label is intended a molecule which provides, directly or indirectly a detectable signal). Various labels may be employed, such as radionuclides, enzymes, fluorescers, chemiluminescers, enzyme substrates, cofactors or inhibitors, particles, e.g., magnetic particles, combinations of ligands and receptors, e.g., biotin and avidin, or the like. In addition the polypeptides may be modified in a variety of ways for binding to a surface, e.g., microwell plate, glass beads, chromatographic surface, e.g., paper, cellulose, silica gel, or the like. The particular manner by which the polypeptides are joined to another compound or surface is conventional and finds ample illustration in the literature. See, for example, U.S. Pat. Nos. 4,371,515; 4,487,715; and patents cited therein.

Various assay protocols may be employed for detecting the presence of either antibodies to retroviral proteins or retroviral proteins themselves. Of particular interest is using the polypeptide as the labeled reagent, where the label allows for a detectable signal, or binding the polypeptide, either directly or indirectly to a surface, where antibody or the polypeptide in the sample will become bound to the polypeptide on the surface. The presence of human antibody bound to the polypeptide can then be detected by employing a xenogeneic antibody specific for human immunoglobulin, normally both human IgM and IgG, or a labeled protein specific for immune complexes, e.g., RF factor or *S. aureus* Protein A.

Various heterogeneous protocols may be employed, either competitive or non-competitive. Polypeptide may be bound to a surface or support ("support") and labeled antibody allowed to compete with antibody in the sample for the limited amount of bound polypeptide. The amount of label bound to the support would be related to the amount of competitive antibody in the sample.

Xenogeneic anti-human antibody, e.g., antibodies to the Fc region of IgG and IgM (immunoglobulins), could be bound to a support. The sample would be contacted with the immunoglobulins and labeled polypeptide, whereby the amount of labeled polypeptide bound to the support would be indicative of the presence of the cognate antibodies.

Alternatively, homogeneous assays can be employed where the polypeptide is bound to an enzyme, fluorescer, or other label, where the binding of antibody to the polypeptide results in being able to discriminate between the label involved with a specific binding pair complex and label which is not involved in the complex. For assays involving such techniques, see for example U.S. Patent Nos. 3,817,837; 3,850,752; 3,901,654; 3,935,074; 3,984,533; 3,996,345; 4,034,074; and 4,098,876.

As an illustration of the subject invention, the subject polypeptide may be conjugated to a fluorescent molecule, such as fluorescein, rhodamine or umbelliferone. In this assay the fluorescence polarization is different between complexed and uncomplexed polypeptide conjugate. Apparatuses are available for measuring changes in fluorescence polarization, e.g., TDx supplied by Abbott Laboratories, Chicago, Illinois.

Illustrative of an assay technique is the use of a sample container, e.g. microwell plate wells, where the subject polypeptide or conjugates thereof are adhered to the container bottom and/or walls either covalently or noncovalently. The sample, normally human blood or serum diluted in appropriately buffered medium, is added to the container and a sufficient time allowed for complex formation between the polypeptide(s) and any cognate antibodies in the sample. The supernatant is removed and the container washed to remove nonspecifically bound proteins.

A labeled specific binding protein which specifically binds to the complex is employed for detection. To the container may be added xenogeneic antisera to human immunoglobulin, particularly anti-(human IgM and IgG) in an appropriately buffered medium. The xenogeneic antisera will normally be labeled with a detectable label, e.g., horseradish peroxidase. The label may then be detected. For example, with an enzyme, after removal of non-specifically bound enzyme label, a developer solution is added. The developer solution will contain an enzyme substrate and possibly enzyme cofactors, chromogens, etc., which, upon reaction, provide a colored, fluorescent, or chemiluminescent product which may be detected colorimetrically, fluorimetrically, or by photon counting, respectively.

The polypeptide can be prepared in a wide variety of ways. The polypeptide, because of its relatively short length, may be synthesized in solution or on a solid support in accordance with known protocols. See, for example, Stewart and Young, Solid Phase polypeptide Synthesis, 2nd ed., Pierce Chemical Co., 1984; and Tam et al, J. Am. Chem. Soc. (1983) 105:6442.

Alternatively, as discussed herein, recombinant DNA technology may be employed where a recombinant polynucleotide sequence may be prepared by employing single strands which code for the polypeptide or substantially complementary strands thereof. (See, for example, Maniatis, *supra*.)

Depending upon the nature of the assay, the physiological sample, e.g., saliva, blood, plasma, or serum, may be pretreated by dilution into an assay medium, which will usually be an aqueous buffered medium employing one of a variety of buffers, such as phosphate, tris, or the like. A preferred diluent is 5% w/v nonfat dry milk, .01% Proclin 300, .005% Antifoam A in 150mM sodium citrate. Usually the pH will be in the range of about 6 to 9. The sample will then be combined with the reagent in accordance with appropriate protocol and sufficient time allowed for binding. Where a heterogeneous system is used, usually the binding stages will be followed by washes to minimize nonspecific binding. At the end of the procedure, the label will be detected in accordance with conventional methods.

Besides the use of the subject polypeptide and its analogs in assays, the subject polypeptide may also find use by itself or in combination in vaccines. The polypeptides may be formulated in a convenient manner, generally at concentrations in the range of 1 ug to 20 mg/kg of host. Physiologically acceptable media may be used as carriers, such as sterile water, saline, phosphate buffered saline, and the like. Adjuvants may be employed, such as aluminum hydroxide gel, or the like. Administration may be by injection, e.g., intramuscularly, intraperitoneally, subcutaneously, intravenously, etc. Administration may be one or a plurality of times, usually at one to four week intervals.

The immunoreactivity of the above-mentioned polypeptides I-IV, which immunologically mimic HIV-1 antigens, to eight known HIV-1 positive sera (i.e., GS91-[034, 037, 042, 046, 049, 052, 056, and 067], 11230, 11424, 11527, 11532 and 11535; all Western-blot all band positives) was examined, and the results are shown in Table 1A of Example 2. All of the polypeptides listed above were highly reactive to those samples.

Analogously, the immunoreactivity of each of the above-mentioned polypeptides, which immunologically mimic HIV-2 antigens, to five known HIV-2 positive sera (i.e., 92099,92100, P-83, P-84, and P-86; Western-blot all band positive) was examined, and the results are shown in Table 1B of Example 2. The glycoprotein (gp) polypeptide, 41-2-3GC, (a nonglycosylated polypeptide was highly reactive to all five samples. All of the polypeptides listed above were reactive to at least two of those five samples. The most reactive polypeptides were ROD 124C2X and ROD 124C5X; they were reactive to all five samples.

### Example 1 - Synthesis of HIV Pol Polypeptides.

Series of HIV pol polypeptides were each synthesized by the sequential coupling of t-butyloxycarbonyl-protected amino acids onto 0.35 mmol p-methylbenzhydrylamine resin (Applied Biosystems Inc., Foster City, CA). Amino acid side chain protection was done by standard benzyl based groups. The tryptophan residue was protected by the formyl moiety. Completed polypeptides were deprotected and cleaved from the resin by the standard high HF procedure or the low-high HF procedure of Tam et al (J. Amer. Chem. Soc. 105:6442, 1983). The cleaved polypeptide was extracted from the resin in 50% acetic acid and subjected to Sephadex G-25 chromatography, using 20% acetic acid as a eluting solvent. Fractions containing polypeptide were pooled and lyophilized.

### Example 2 - Immunoreactivity of Pol Polypeptides:

Polypeptides of the invention were tested for immunological reactivity by ELISA as previously described in U.S. Patent No. 4,629,783. Briefly, stock solutions of polypeptides of the invention at 0.5mg/ml were prepared in 2M urea/5% acetic acid. Twelve milliters of 1.2% acetic acid was placed in a 15 milliliter polypropylene tube and 48 microliters of the polypeptide stock solution added to the tube and mixed (the "coating solution"). Wells of microwell plates were filled with 100ul of the coating solution of the polypeptides and 100ul/well of 0.24M carbonate/0.2N NaOH added to raise the coating solution to an alkaline pH. The plate was covered and allowed to stand overnight at room temperature. The coating solution was removed by aspiration and 300ul/well of Plate Blocking solution (containing per liter; 25g non-fat dry milk, 14.7g sodium citrate dihydrate, 8.47g sodium chloride and 0.05ml Antifoam A, 1.0 ml Kathon GC/ICP) was added and incubated for 1 hr. at room temperature. Blocking solution was removed by aspiration, and the plates were used immediately or allowed to air-dry and stored for later use. To carry out the immunoassay, plasma samples were diluted 20-fold in Specimen Diluent (containing per liter; 44.1g sodium citrate dihydrate, 1.2m1 Tween 20, 50g non-fat dry milk, 0.05ml Antifoam A, 50ml goat serum, 58.6g 2-[N-morpholino]ethane sulfonic acid, 92.9g triethanolamine hydrochloride, 1ml Proclin 300) and 100ul was added to individual wells. Samples were incubated for 30 minutes at 37° C, then removed and the wells were washed five times with 0.1M NaCl/0.05% Tween 20 (350ul/wash). One hundred microliters of goat antihuman Ig-horseradish peroxidase conjugate diluted in citrate buffer, pH 7.0, containing 1% normal goat serum was added to each well for 30 minutes at 37° C prior to washing five times as above. The ELISA assay was developed by adding 100ul/well of substrate solution (80ug/ml tetramethylbenzidine, 0.0015% hydrogen peroxide in citrate/phosphate buffer, pH 6.0) for 30 minutes at room temperature. Reactions were stopped with the addition of 100ul of 1N H₂SO₄ per well, and the ratio of the optical density at 450 nm to 630 nm was determined by an automated ELISA reader. The cut-off value for a positive result was set at 0.200 Absorbance Units above the average absorbance obtained from at least three known negative samples.

The results in Table 1A shows the reactivity of the polypeptides with HIV-1 positive and negative samples wherein the HIV-1 positive samples are GS91-(034, 037, 042, 046, 049, 052, 056, 067), 11230, 11424, 11527, 11532 and 11535, and the negative samples are PS1059-PS1062, PS1068, PS1071, and D21-D25.

**TABLE 1A**

| Absorbance (450nm/630nm)** | | | | |
|---|---|---|---|---|
| Sample* | BRU124E | BRU124F1X | BRU124F3X | BRU124EX*** |
| GS91-034 | **0.606** | **1.000** | **0.945** | n.d. |
| GS91-037 | **0.507** | **1.584** | **1.551** | n.d. |
| GS91-042 | **1.860** | **1.899** | **1.888** | n.d. |
| GS91-046 | **1.598** | **1.848** | **1.831** | n.d. |
| GS91-049 | **1.034** | **1.955** | **1.991** | n.d. |
| GS91-052 | **1.606** | **1.852** | **1.917** | n.d. |
| GS91-056 | **1.848** | **1.966** | **1.957** | n.d. |
| GS91-067 | **1.960** | **2.110** | **2.110** | n.d. |
| PS1071 | 0.036 | 0.040 | 0.046 | n.d. |
| PS1062 | 0.041 | 0.047 | 0.046 | n.d. |
| PS1070 | 0.034 | 0.039 | 0.043 | n.d. |
| PS1061 | 0.020 | 0.023 | 0.021 | n.d. |
| PS1069 | 0.034 | 0.039 | 0.039 | n.d. |
| PS1060 | 0.027 | 0.052 | 0.044 | n.d. |
| PS1068 | 0.028 | 0.039 | 0.037 | n.d. |
| PS1059 | 0.043 | 0.046 | 0.049 | n.d. |
| | | | | |
| 11535 | n.d. | n.d. | n.d. | **1.652** |
| 11527 | n.d. | n.d. | n.d. | **2.595** |
| 11532 | n.d. | n.d. | n.d | **2.912** |
| 11424 | n.d. | n.d. | n.d. | **2.676** |
| 11230 | n.d. | n.d. | n.d | **0.759** |
| D21 | n.d. | n.d. | n.d. | 0.096 |
| D22 | n.d. | n.d. | n.d. | 0.045 |
| D23 | n.d. | n.d. | n.d. | 0.053 |
| D24 | n.d. | n.d. | n.d. | 0.044 |
| D25 | n.d. | n.d. | n.d | 0.034 |

| | | | | |
|---|---|---|---|---|
| * Samples were diluted 1/40, instead of 1/20, in order to be able to compare the absorbance between different polypeptides. The GS91- (034, 037, 042, 046, 049, 052, 056, 067), 11230, 11424, 11527, 11532, and 11535) are known HIV-1 positive samples; samples PS1059-PS1062, PS1068, PS1071, and D21-25 are known HIV-1 negative samples. ** Highlighted values are positive values based on the cut-off values established by 0.200 + average negative. Cut-Off = 0.238 (except BRU124EX) The Cut-Off value for BRU124EX = 0.254 ***The testing for the reactivity of BRU124EX was done separately at different date, using different samples. n.d. = not done (or tested) | | | | |

The results in Table 1B show the reactivity of the polypeptides of the invention with HIV-2 positive and negative samples wherein the HIV-2 positive samples are 92099, 92100, P-83, P-84 and P-86 and the negative samples are NBD1, NBD2, NBD3, AND NBD4.

**TABLE 1B**

| Absorbance (450nm/630nm)* | | | | | | | | |
|---|---|---|---|---|---|---|---|---|
| Sample | 41-2-3GC | E1 | EX | C1X | C2X | C3X | C5X | 2A1 |
| 92099 | **>3.00** | **0.41** | **0.73** | **0.24** | **1.61** | **0.78** | **1.77** | **1.91** |
| 92100 | **>3,00** | **0.47** | **0.75** | **0.26** | **1.91** | **0.88** | **1.82** | **1.86** |
| P-83 | **2.82** | **0.44** | **1.05** | 0.10 | **1.02** | **0.45** | **1.06** | 0.08 |
| P-84 | **>3.00** | **0.47** | **1.04** | **0.27** | **0.75** | **0.40** | **0.73** | 0.18 |
| P-86 | **>3.00** | 0.16 | 0.23 | 0.16 | **0.35** | 0.20 | **0.34** | 0.13 |
| NBD1 | 0.07 | 0.06 | 0.06 | 0.05 | 0.05 | 0.05 | 0.05 | 0.09 |
| NBD2 | 0.11 | 0.11 | 0.12 | 0.11 | 0.11 | 0.11 | 0.12 | 0.11 |
| NBD3 | 0.04 | 0.03 | 0.05 | 0.03 | 0.03 | 0.05 | 0.03 | 0.03 |
| NBD4 | 0.07 | 0.05 | 0.06 | 0.05 | 0.04 | 0.05 | 0.07 | 0.05 |

| | | | | | | | | |
|---|---|---|---|---|---|---|---|---|
| Highlighted values are positive values based on the cut-off values established by 0.200 + average Negative. Cut-Off = 0.256 Known positive samples are 92099, 92100, P-83, P-84 and P-86 and the known negative samples are NBD1, NBD2, NBD3, AND NBD4. | | | | | | | | |

An improvement on the specificity of HIV-1 and HIV-2 antibody detection by the incorporation of pol polypeptides (BRU124F3X and ROD124C5X) is illustrated by the study results shown in Table 1C. In this study, the pol polypeptides were coated on the microwell plate individually or together with envelope specific polypeptides, as mentioned in U.S. Patent No. 5,439,792. For the plate coating with individual pol polypeptides, the polypeptide was coated at 1.0 ug/ml. For the plate coating of the mixture of HIV-1 and HIV-2 polypeptides, the polypeptides were mixed together at the following concentrations in the coating buffer: 1.23 ug/ml for HIV-1 envelope polypeptide (designated as MNGC), 0.64 ug/ml for HIV-2 envelope polypeptide (designated as 41-2-3GC), 0.25 ug/ml for BRU124F3X and 0.125 ug/ml for ROD124C5X. The polypeptide coating procedure was the same as described earlier in the "immunoreactivity" section. The samples tested are known HIV-1 (Western blot all-band positive) samples (i.e., SAL040, SAL041, SAL059, SAL063, SAL064), known HIV-2 (Western blot all-band positive) samples (i.e., 52, GB92000128, GB92000152, GB92000154, GB92000158), HIV-1 indeterminate samples (i.e., B3113, B5813, B5885, B7045, C000127, C000214, C000455) and HIV-2 indeterminate samples (i.e., B3123, B5605, 85810, B5826, B5832, B5875, B6312). Also included are the control samples used in the Genetic Systems® HIV-1/HIV-2 Peptide EIA kit (available from Sanofi Diagnostics Pasteur, Inc., Redmond, Washington), namely HIV-1 positive control (PC-1), HIV-2 positive control (PC-2) and negative control (NC). Both the known positive and the indeterminate samples were also tested on a commercially available viral lysate based test, the Genetic Systems® HIV-1/HIV-2 EIA (Sanofi Diagnostics Pasteur, Inc., Redmond, Washington).

**TABLE 1C**

| **COMPARISON OF HIV-1/2 WESTERN BLOT ALL BAND POSITIVE AND INDETERMINATE SAMPLE REACTIVITIES IN ELISA WITH POL ONLY COATED PLATES AND FOUR PEPTIDE COATED PLATES** | | | | | | | | | | | | |
|---|---|---|---|---|---|---|---|---|---|---|---|---|
| Sample | 4 Peptides* | | | HIV-1 pol(BRU124F3X) Only | | | HIV-2 pol (ROD124C5X) Only | | | | HIV-1/2 Viral Lysate*** | |
| | A450/A630 | S/CO | Pos/Neg** | A450/A630 | S/CO | Pos/Neg** | A450/A630 | S/CO | Pos/Neg** | | S/CO | Pos/Neg** |
| PC-1 | 1.610 | 4.8 | P | 1.503 | 3.0 | P | 0.441 | 0.8 | N | | N.A. | N.A. |
| | 1.639 | 4.9 | P | 1.539 | 3.1 | P | 0.451 | 0.9 | N | | N.A. | N.A. |
| PC-2 | 1.259 | 3.8 | P | 0.307 | 0.6 | N | 0.438 | 0.8 | N | | N.A. | N.A. |
| | 1.221 | 3.7 | P | 0.301 | 0.6 | N | 0.560 | 1.1 | P | | N.A. | N.A. |
| NC | 0.093 | 0.3 | N | 0.254 | 0.5 | N | 0.279 | 0.5 | N | | N.A. | N.A. |
| | 0.091 | 0.3 | N | 0.265 | 0.5 | N | 0.302 | 0.6 | N | | N.A. | N.A. |
| Cut-off | 0.332 | N.A. | | 0.500 | N.A. | | 0.531 | N.A. | | | N.A. | N.A. |
| | | | | | | | | | | | | |
| HIV-1 WB Pos. | | | | | | | | | | | | |
| SAL040 | 2.985 | 9.0 | P | 2.956 | 5.9 | P | 2.709 | 5.1 | P | | >8.0 | P |
| SAL041 | 3.028 | 9.1 | P | 2.876 | 5.8 | P | 2.555 | 4.8 | P | | >8.0 | P |
| SAL059 | 2.957 | 8.9 | P | 2.796 | 5.6 | P | 2.238 | 4.2 | P | | >8.0 | P |
| SAL063 | 3.009 | 9.1 | P | 2.790 | 5.6 | P | 2.714 | 5.1 | P | | >8.0 | P |
| SAL064 | 2.934 | 8.8 | P | 2.893 | 5.8 | P | 2.865 | 5.4 | P | | >8.0 | P |
| HIV-2 WB Pos. | | | | | | | | | | | | |
| 52 | 2.972 | 9.0 | P | 2.738 | 5.5 | P | 2.669 | 5.0 | P | | >8.0 | P |
| GB92000128 | 2.939 | 8.9 | P | 2.698 | 5.4 | P | 2.872 | 5.4 | P | | >8.0 | P |
| GB92000152 | 2.904 | 8.7 | P | 2.633 | 5.3 | P | 2.743 | 5.2 | P | | >8.0 | P |
| GB92000154 | 3.011 | 9.1 | P | 2.677 | 5.4 | P | 2.857 | 5.4 | P | | >8.0 | P |
| GB92000158 | 3.061 | 9.2 | P | 2.723 | 5.5 | P | 2.930 | 5.5 | P | | >8.0 | P |
| HIV-1 Indeterminates | | | | | | | | | | | | |
| B3113 | 0.094 | 0.3 | N | 0.346 | 0.7 | N | 0.253 | 0.5 | N | | **3.2** | **FP** |
| B5813 | 0.063 | 0.2 | N | 0.303 | 0.6 | N | 0.244 | 0.5 | N | | **3.0** | **FP** |
| B5885 | 0.063 | 0.2 | N | 0.152 | 0.3 | N | 0.154 | 0.3 | N | | **3.1** | **FP** |
| B7045 | 0.062 | 0.2 | N | 0.241 | 0.5 | N | 0.208 | 0.4 | N | | **3.5** | **FP** |
| C000127 | 0.050 | 0.2 | N | 0.172 | 0.3 | N | 0.177 | 0.3 | N | | **3.1** | **FP** |
| C000214 | 0.056 | 0.2 | N | 0.157 | 0.3 | N | 0.155 | 0.3 | N | | **5.4** | **FP** |
| C000455 | 0.065 | 0.2 | N | 0.136 | 0.3 | N | 0.179 | 0.3 | N | | **5.4** | **FP** |
| HIV-2 Indeterminates | | | | | | | | | | | | |
| B3123 | 0.051 | 0.2 | N | 0.142 | 0.3 | N | 0.188 | 0.4 | N | | **3.2** | **FP** |
| B5805 | 0.074 | 0.2 | N | 0.203 | 0.4 | N | 0.276 | 0.5 | N | | **3.8** | **FP** |
| B5810 | 0.053 | 0.2 | N | 0.204 | 0.4 | N | 0.205 | 0.4 | N | | **4.3** | **FP** |
| B5826 | 0.049 | 0.1 | N | 0.145 | 0.3 | N | 0.177 | 0.3 | N | | **3.0** | **FP** |
| B5832 | 0.057 | 0.2 | N | 0.183 | 0.4 | N | 0.195 | 0.4 | N | | **3.2** | **FP** |
| B5875 | 0.056 | 0.2 | N | 0.254 | 0.5 | N | 0.286 | 0.5 | N | | **3.8** | **FP** |
| B6312 | 0.027 | 0.1 | N | 0.150 | 0.3 | N | 0.155 | 0.3 | N | | **2.8** | **FP** |

| | | | | | | | | | | | | |
|---|---|---|---|---|---|---|---|---|---|---|---|---|
| * 4 peptides = MNGC (1.23ug/ml): 41-2-3GC (0.64ug/ml): BRU124F3X (0.25ug/ml): ROD124C5X (0.125ug/ml) HIV-1pol (BRU124F3X) Only and HIV-2 pol (124C5X) Only = 1.0ug/ml each HIV-1 and HIV-2 WB Pos. = All band Western blot Positive samples HIV-1 and HIV-2 indeterminates = Western blot Indeterminates ** In Pos./Neg. columns : P = Positive; N = Negative; FP = False Positive *** Genetic Systems® HIV-1/2 EIA (FDA approved) | | | | | | | | | | | | |

Table 1C shows that all of the known HIV-1 and HIV-2 positive samples showed positive results when using either a single pol polypeptide coated plate or the plate coated with all four polypeptides. All HIV-1 and HIV-2 indeterminate samples showed negative results when using either a single pol polypeptide coated plate or the plate coated with all four polypeptides. The indeterminate samples showed highly positive results (false positive) when tested using the viral lysate-based HIV-1/HIV-2 EIA. These results very clearly show that the polypeptide based EIA incorporating the pol polypeptides of the invention is highly sensitive and specific in detecting HIV positive samples.

### Example 3 - Cloning pol23 and pol7:

Both *pol*23 and *pol*7 recombinants were initially cloned in the pGEX system (Pharmacia). The cloned inserts were then moved into other expression vectors that provide "purification handles" to simplify analysis of gene expression.

### Recombinant: pGEX/pol23

A PCR product was generated using HIV-1 viral DNA isolated from HIV-1 LAI strain viral lysate as a template, and the primer pair 5' AGCACCATGGGGATCCCAGGGATTAGATATCAGTACAATG 3' and 5' AGTCAGAATTCATTGGCCTTGCCCCTGCTT 3'. PCR reactions used *UlT*ma DNA polymerase (Perkin-Elmer) to minimize the possibility of mutation due to polymerase error. The PCR product was digested with the restriction enzymes *Bam*HI and *Eco*RI and inserted into *Bam*HI, *Eco*RI-digested pGEX 5X-1 (Pharmacia). The ligation mix was transformed into *E*. *coli* DH11S and ampicillin-resistant colonies selected (see Maniatis, *supra*).

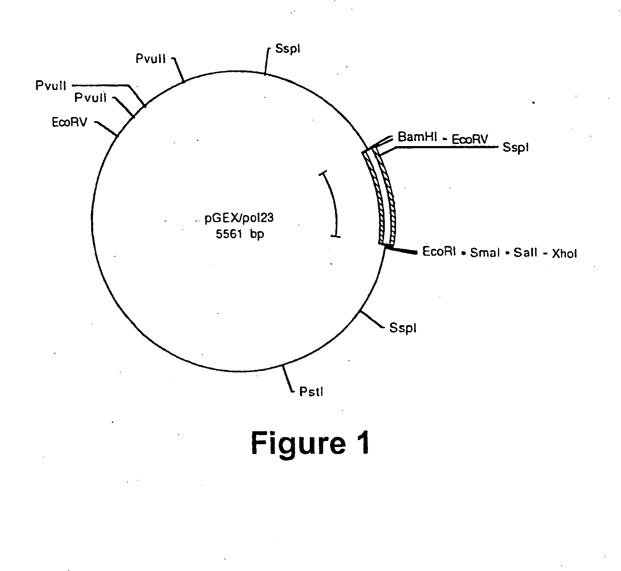

Insert-containing colonies were initially identified by colony-PCR. This was followed by growth, plasmid isolation¹, and restriction analysis to demonstrate that the candidate clones contained the predicted restriction sites (see Maniatis, *supra*). A plasmid map of the resulting clone is provided in Figure 1.
¹ Plasmid DNA was prepared from recombinant bacterial cultures grown overnight at 37°C in L-broth plus 100mg/ml ampicillin using the QIAwell 8 Plus Plasmid Kit (QIAGEN).

### Recombinant: pGEX/pol7.0

As described above, a PCR product was generated using a HIV-1 viral DNA template, and the primer pair 5' AGCACCATGGGGATCCCCTACAGTGCAGGGGAAAGAATA 3' and 5' GACTAGTCGACTCAATCATCACCTGCCATCTG 3'. PCR reactions used *UlT*ma DNA polymerase (Perkin-Elmer) to minimize the possibility of mutation due to polymerase error. The PCR product was digested with the restriction enzymes *Bam*HI and *Sal*I and inserted into *Bam*HI*, Sal*I I-digested pGEX 5X-1. The ligation mix was transformed into *E. coli* DH11S and ampicillin-resistant colonies selected.

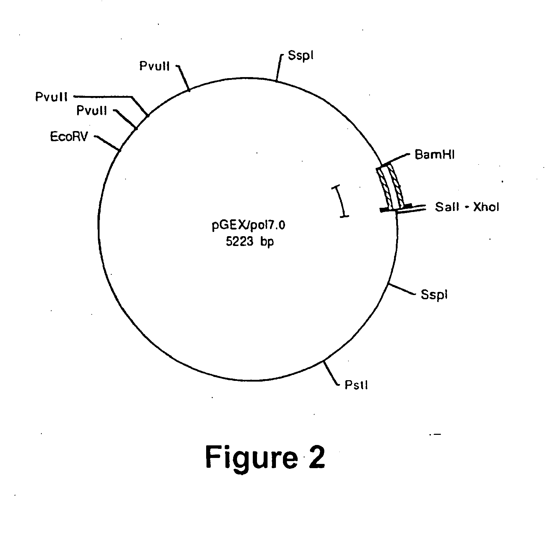

Insert-containing colonies were initially identified by colony-PCR. This was followed by growth, plasmid isolation, and restriction analysis to demonstrate that the candidate clones contained the predicted restriction sites. A plasmid map of the resulting clone is provided in Figure 2.

### Subcloning of pGEX/pol23 and pGEX/pol7.0 into expression vectors pQE42 and pThioHisA

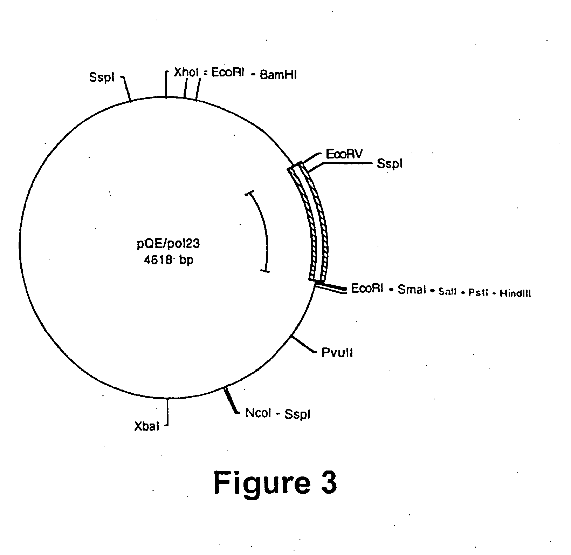

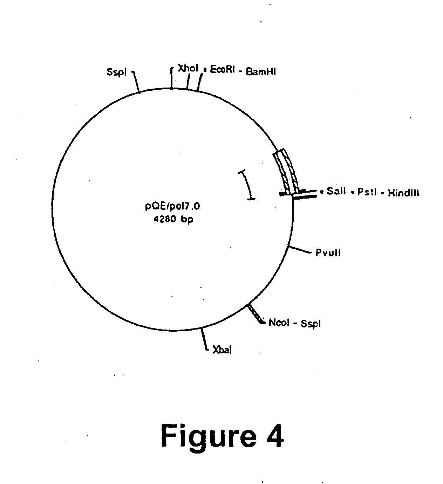

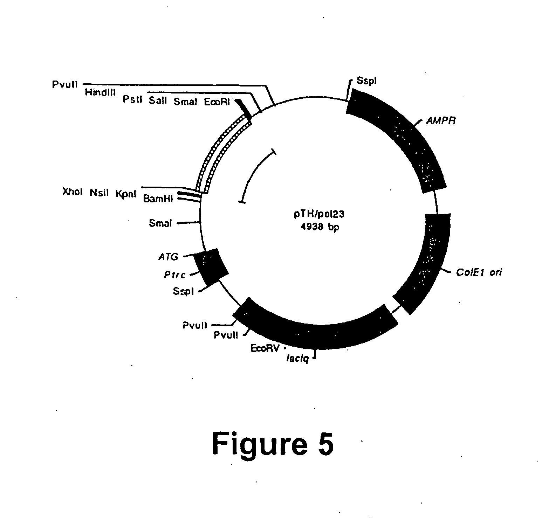

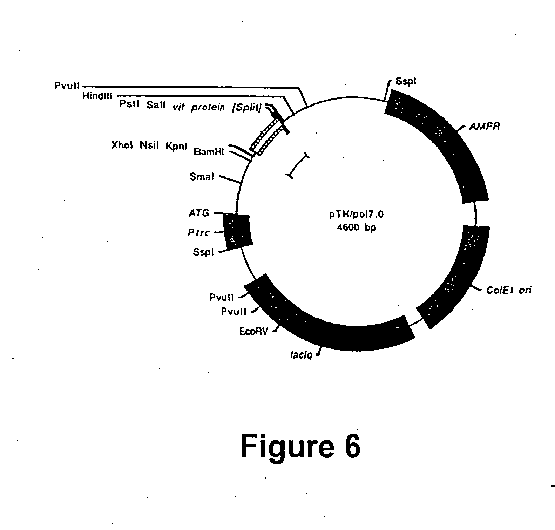

Insert DNA was prepared from pGEX/*pol*23 and pGEX/*pol*7.0 by digestion with the enzymes *Bam*HI and *Sal*I. The commercial expression vectors pThioHisA (Invitrogen) and pQE42 (QIAGEN) were each digested with the enzymes *Bgl*II² and *Sal*I. In separate ligation reactions, each insert was joined to each vector. (see Maniatis, *supra*) Again, ampicillin-resistant transformants were selected in the *E. coli* host strain DH11S. Identity of the clones was verified by colony-PCR and restriction analysis of purified plasmid DNA. The resulting recombinants were designated pQE/*pol*23, pQE/*pol*7.0, pThioHis/*pol*23, and pThioHis/*pol*7.0. (Plasmid maps of the resulting clones are provided in Figures 3 through 6 respectively).
² Note that the enzymes *Bam*HI and *Bgl*II produce cohesive ends that can be joined by ligation.

### Example 4 - Protein Expression of Example 3 Clones:

All three plasmid expression systems, pGEX, pThioHisA, and pQE42, employing the *tac* promoter are negatively regulated (shut off) by glucose, and positively regulated (turned on) by the lactose analog IPTG.

Twenty-five ml cultures of each of the recombinants pQE/*pol*23, pQE/*pol*7.0, pThioHis/*pol*23, and pThioHis/*pol*7.0 were grown in complete T-broth plus 100mg/ml ampicillin, 1.0% glucose at 37°C (with shaking) until the optical density at 600 nm was between 2 and 6. Cells were then pelleted and resuspended in fresh glucose-free medium that contained 1mM IPTG to induce expression of the recombinant fusion protein. After 2 additional hours of incubation, the induced cultures were harvested by centrifugation at 2000 rpm for 10 minutes. The resulting supernatant medium was discarded and the cell pellets frozen at -70°C.

Frozen pellets were thawed and resuspended in 2.0 ml of an aqueous medium of 20 mM sodium phosphate, pH 7.8 and 500 mM NaCl. The cells were lysed by two cycles of freezing in a dry ice/ethanol bath followed by thawing in warm water and 10 short bursts of sonication. The resulting lysate was then centrifuged in an Eppendorf microfuge at maximum speed until pelleted. The lysate supernatants and pellets were used for further purification.

### pQE/pol23, pQE/pol7.0: Ni-NTA purification

For these recombinants, the lysate pellets were dissolved in an aqueous buffer comprising 20 mM sodium phosphate, pH 7.8 and 8M urea. This suspension was bound to Ni-NTA agarose (QIAGEN) that was pre-equilibrated in the same buffer. Unbound material was washed away using the same buffer. Bound protein was then eluted with an aqueous buffer comprising 20 mM sodium phosphate, 6.4 M urea and 100 mM EDTA. The resulting purified fusion proteins were analyzed by SDS-PAGE.

The lysate supernatants were also purified, using methods found below, however higher levels of recombinant protein were found in the lysate pellets.

### pThioHis/pol23, pThioHis/pol7.0: Ni-NTA purification

For these recombinants, lysate supernatants were bound to Ni-NTA that was pre-equilibrated in an aqueous buffer comprising 20 mM sodium phosphate, pH 7.8 and 500 mM NaCl. Unbound material was washed away with the same buffer, and then again with an aqueous wash buffer (20 mM sodium phosphate, pH 6.0, 500 mM NaCl).

Bound protein was eluted in four successive steps using increasing concentration of imidazole in the wash buffer (50 mM, 200 mM, 350 mM, 500 mM). SDS-PAGE analysis showed that 350 mM imidazole was the optimal elution condition for these recombinants.

### SDS-PAGE and Western Blot Analysis

Using standard laboratory procedures, samples of the resulting partially purified fusion proteins were separated by SDS-Page. Novex 4-20% polyacrylamide gradient gels were used.

In all cases it was observed that the recombinant fusion proteins migrated close to the predicted sizes as found in Table 2.

**TABLE 2**

| **Recombinant** | **Predicted fusion protein molecular weight** | **Purified from** |
|---|---|---|
| pQE/pol23 | 46 kD | lysate pellet |
| pQE/pol7.0 | 33.5 kD | lysate pellet |
| pThioHis/pol23 | 37 kD | lysate supernatant |
| pThioHis/pol7.0 | 24 kD | lysate supernatant |

The separated proteins were also transferred to a nitrocellulose support for Western blot analysis. The semi-dry electrophoretic transfer method (Harlow and Lane, Antibodies, a laboratory manual pp. 488-489) was used to transfer electrophoresed proteins to nitrocellulose membranes.

After transfer, membranes were blocked with Blotto (50 mM Tris pH7.5, 150 mM NaCl, 5.0% nonfat dry milk, 0.1 % Tween-20) either overnight at 4°C or one hour at room temperature. After removing the blocking agent, the membrane was incubated with the primary antibody³ diluted in Blotto, for one hour at room temperature. The primary antibody solution is then discarded and the blot is washed four times for 5 minutes each
³ Two mouse monoclonal antibodies against HIV *pol* were used at 1:4000 dilutions. A human serum positive to HIV was used at a 1:500 dilution. with Blotto. The secondary antibody-enzyme conjugate⁴ is then added for a 30 minute room temperature incubation. After 30 minutes, the secondary antibody is discarded and the blot is again washed four times as earlier. This is followed by a rinse with water and then addition of the color substrate solution⁵.
⁴ The secondary antibody conjugate was either horseradish peroxidase-conjugated goat-antimouse antibody (1:2000) in Blotto if the primary antibody was mouse monoclonal or alkaline phosphatase-conjugated goat-antihuman antibody (1:2000) in Blotto. if the primary antibody was human serum.
⁵ Tetramethylbenzidine (TMB) (Vector Labs) was the substrate used with the horseradish peroxidase secondary antibody conjugate, while BCIP/NBT One-Component Substrate (Kirkegaard & Perry Laboratories Inc.) was used with the alkaline phosphatase secondary antibody conjugate.

Color development was stopped by washing with water after bands appeared. The pQE/*pol*23 and pThioHis/*pol*23 protein bands both reacted in Western blots using one of the mouse monoclonal antibodies, while the pQE/*pol*7.0 and pThioHis/*pol*7.0 protein bands both reacted with the other . All four band were detected with the human HIV-1 positive serum.

### Example 5 - Enzyme Immunoassay (EIA) Testing:

EIA plates were coated with the partially purified recombinant fusion proteins. Each sample was coated at a dilution of 1:500 in 15 mMcarbonate/35 mM bicarbonate coating buffer pH 9.6 overnight at room temperature. The coated plates were then "ON/OFF" blocked (2.5% non-fat dry milk), "ON/OFF" coated with a 4% sucrose-solution, and air-dried overnight. As prepared, the plates may be stored in a sealed pouch with desiccant.

Each recombinant fusion protein was tested for reactivity with 16 HIV-1 positive samples, 8 normal donor samples, and 4 *E. coli*-reactive samples. The *E. coli*-reactive samples are samples that have previously been shown to have high reactivity to proteins from *E*. *coli* lysate. These were included to ensure that the reactivity we observe with HIV-1-positive samples is specific to the recombinant polypeptide and not directed toward contaminating *E. coli* proteins.

Briefly, the samples were diluted 1:101 in a specimen diluent (containing per liter; 44.1g sodium citrate dihydrate, 1.2ml Tween 20, 50g non-fat dry milk, 0.05ml Antifoam A, 50ml goat serum, 58.6g 2-[N-morpholino]ethane sulfonic acid, 92.9g triethanolamine hydrochloride, 1ml Proclin 300) and 200 µl of the resulting mixtures were added to each well of the previously prepared plates. The plates were covered and incubated at 37 ± 1° C for 60 minutes. The fluid was aspirated from each well and the plate was washed a minimum of 5 times with a wash solution (0.1M NaCl/0.05% Tween 20).

100 µl of a conjugate solution (goat antihuman Ig-horseradish peroxidase conjugate diluted in citrate buffer, pH 7.0, containing 1% normal goat serum) was added to each well. The plates were then covered and incubated for 60 minutes at 37 ± 1° C. Following incubation, the fluid was aspirated from each well and the plate was washed a minimum of 5 times with the wash solution.

100 µl of a chromogen solution (80ug/ml tetramethylbenzidine, 0.0015% hydrogen peroxide in citrate/phosphate buffer, pH 6.0) was added to each well. The plates were then covered and incubated in the dark for 30 minutes at room temperature. After removing the cover and adding 100 µl of a stopping reagent (1N H₂SO₄) the plates were read at an absorbance of 450 nm with 615 nm to 630 nm as a reference. The ratio of the optical densities at 450 nm to 630 nm was calculated. The cut-off value for a positive result was set at 0.200 Absorbance Unites above the average absorbance obtained from at least three known negative samples. The results of these procedures are presented in Table 3.

**TABLE 3**

| | **Coated Plates** | | | |
|---|---|---|---|---|
| ***Samples*** | ***pQE*/*pol23*** | ***pQE*/*pol7.0*** | ***pTH*/*pol23*** | ***pTH*/*pol7.0*** |
| ***B7095*** | 2.122 | 1.903 | 0.557 | 1.68 |
| ***C000218*** | 1.553 | 2.11 | 0.046 | 2.042 |
| ***C000582*** | 1.557 | 1.618 | 0.208 | 1.219 |
| ***B3137*** | 2.075 | 2.155 | 0.949 | 1.933 |
| ***B5878*** | 2.142 | 0.107 | 0.732 | 0.064 |
| ***B5879*** | 2.255 | 2.13 | 1.075 | 2.14 |
| ***B5847*** | 0.042 | 0.132 | 0.01 | 0.049 |
| ***C000024*** | 0.051 | 2.278 | 0.018 | 2.225 |
| ***B5853*** | 1.671 | 2.212 | 0.509 | 2.154 |
| ***B3138*** | 0.916 | 1.732 | 0.2 | 1.295 |
| ***B5895*** | 0.944 | 0.717 | 0.027 | 0.587 |
| ***C000130*** | 0.438 | 0.598 | 0.021 | 0.299 |
| ***C000136*** | 0.947 | 1.622 | 0.08 | 1.385 |
| ***B5889*** | 1.438 | 1.139 | 0.25 | 0.649 |
| ***B5822*** | 0.331 | 0.05 | 0.051 | 0.031 |
| ***SAL054*** | 2.044 | 1.802 | 0.955 | 1.771 |
| | | | | |
| ***PSBC1171*** | 0.07 | 0.021 | 0.019 | 0.014 |
| ***PSBC1265*** | 0.033 | 0.019 | 0.014 | 0.018 |
| ***PSBC1341*** | 0.014 | 0.01 | 0.009 | 0.01 |
| ***PSBC1398*** | 0.028 | 0.015 | 0.013 | 0.013 |
| ***PSBC1481*** | 0.027 | 0.017 | 0.014 | 0.016 |
| ***PSBC1521*** | 0.021 | 0.023 | 0.013 | 0.014 |
| ***PSBC1657*** | 0.028 | 0.017 | 0.014 | 0.011 |
| ***PSBC1566*** | 0.017 | 0.012 | 0.011 | 0.01 |
| | | | | |
| ***EC*+*1*** | 0.039 | 0.028 | 0.015 | 0.025 |
| ***EC*+*2*** | 0.046 | 0.074 | 0.021 | 0.03 |
| ***EC*+*3*** | 0.048 | 0.024 | 0.014 | 0.03 |
| ***EC*+*4*** | 0.022 | 0.015 | 0.011 | 0.023 |

It is evident from the foregoing results that by employing one or a combination of polypeptides of the subject invention, a sensitive, accurate test for the presence of antibodies to HIV is provided. The subject polypeptides can be used by themselves or in combination with a screening assay or confirmatory assay, whereas the complete lysate or complete antigens may be employed as an independent procedure. The subject polypeptides can also be combined with polypeptides or proteins derived from the envelope or gag regions of HIV-1 or HIV-2 in a screening assay or confirmatory assay. Furthermore, because of the specificities of the polypeptides, one could anticipate that the DNA sequences coding for the polypeptides would also find similar specificity in a DNA hybridization assay.

## Claims

1. A method for determining the presence of antibodies to HIV in a body fluid, comprising:
(a) contacting under conditions which permit immunospecific binding to form a reaction mixture the body fluid with a composition containing at least one polypeptide selected from the group consisting of the amino acid sequences:
(V) ROD 124 E1 
W-X-Lys-Leu-Lys-Asp-Phe-Arg-Val-Tyr-Phe-Arg-Glu-Gly-
Arg-Asp-Gln-Leu-Trp-Lys-Gly-Pro-Gly-Glu-Leu-Leu-Trp-
Lys-Gly-Glu-Gly-Ala-Y-Z
(VII) ROD 124C2X 
W-X-Lys-Leu-Lys-Asp-Phe-Arg-Val-Tyr-Phe-Arg-Glu-Gly-
Arg-Asp-Gln-Leu-Trp-Lys-Gly-Pro-Gly-Glu-Leu-Leu-Trp-
Lys-Gly-Glu-Gly-Ala-Val-Leu-Val-Lys-Val-Gly-Thr-Asp-
Ile-Lys-Y-Z
(VIII) ROD C1X 
W-X-Tyr-Phe-Arg-Glu-Gly-Arg-Asp-Gln-Leu-Trp-Lys-Gly-
Pro-Gly-Glu-Leu-Leu-Trp-Lys-Gly-Glu-Gly-Ala-Val-Leu-
Val-Lys-Val-Gly-Thr-Asp-Ile-Lys-Y-Z
(IX) ROD C3X 
X-Lys-Leu-Lys-Asp-Phe-Arg-Val-Tyr-Phe-Arg-Glu-Gly-
Arg-Asp-Gln-Leu-Trp-Lys-Gly-Pro-Gly-Glu-Leu-Leu-Trp-
Lys-Gly-Glu-Gly-Ala-Val-Leu-Val-Lys-Val-Gly-Thr-Asp
Ile-Lys-Y-Z
(XI) ROD 124C5X 
W-X-Lys-Leu-Lys-Asp-Phe-Arg-Val-Tyr-Phe-Arg-Glu-Gly-
Arg-Asp-Gln-Leu-Trp-Lys-Gly-Pro-Gly-Glu-Leu-Leu-Trp-
Lys-Gly-Glu-Gly-Als-Val-Leu-Val-Lys-Val-Gly-Y-Z
wherein W is either a H of the amino terminal NH₂ group of the polypeptide or one or more additional amino acids bonded to the amino terminal NH₂ group of the polypeptide, the additional amino acids being selected to facilitate coupling of the polypeptide to a carrier protein or to a support; X is absent, Cys-Gly-Gly; or Lys-Lys, Y is absent, Cys- or one or more amino acids added to facilitate coupling; and Z is OH or NH;
(b) detecting whether immunospecific binding has occurred between the polypeptide and an antibody component of the body fluid in which an immune complex is formed and in which the detection of the immune complex indicates the presence of antibodies to HIV in the body fluid.

2. The method according to claim 1 in which the polypeptide is conjugated to a carrier macromolecule.

3. The method according to claim 1 in which the polypeptide is immobilized.

4. The method according to claim 1 in which the immunospecific binding is detected by immunoprecipitation.

5. The method according to claim 1 in which the composition includes at least one polypeptide selected from a polymerase protein of HIV-2 and one selected from a polymerase protein of HIV-1.

6. The method according to claim 1 in which immunospecific binding between the polypeptide or protein and the antibody component of the body fluid is detected by:
(i) removing unbound component from immune complexes formed in the immunoreaction mixture;
(ii) adding a labeled antibody to the immunoreaction mixture, the labeled antibody being capable of immunospecifically binding to a component of the immune complexes and the label providing a detectable signal; and
(iii) determining whether the labeled antibody binds to the immune complexes.

7. The method according to claim 6 in which the label comprises an enzyme which is detected by the addition of the enzyme substrate, a radiolabel, or a fluorescent label.

8. The method for determining the presence of antibodies to HIV-2 in a body fluid, comprising:
(a) contacting under conditions which permit immunospecific binding to form a reaction mixture the body fluid with a composition containing at least one polypeptide selected from the group consisting of the following amino acid sequences:
(V) ROD 124 E1 
W-X-Lys-Leu-Lys-Asp-Phe-Arg-Val-Tyr-Phe-Arg-Glu-Gly-
Arg-Asp-Gln-Leu-Trp-Lys-Gly-Pro-Gly-Glu-Leu-Leu-Trp-
Lys-Gly-Glu-Gly-Ala-Y-Z
(VII) ROD 124C2X 
W-X-Lys-Leu-Lys-Asp-Phe-Arg-Val-Tyr-Phe-Arg-Glu-Gly-
Arg-Asp-Gln-Leu-Trp-Lys-Gly-Pro-Gly-Glu-Leu-Leu-Trp-
Lys-Gly-Glu-Gly-Ala-Val-Leu-Val-Lys-Val-Gly-Thr-Asp-
Ile-Lys-Y-Z
(VIII) ROD C1X 
W-X-Tyr-Phe-Arg-Glu-Gly-Arg-Asp-Gln-Leu-Trp-Lys-Gly-
Pro-Gly-Glu-Leu-Leu-Trp-Lys-Gly-Glu-Gly-Ala-Val-Leu-
Val-Lys-Val-Gly-Thr-Asp-Ile-Lys-Y-Z
(IX) ROD C3X 
X-Lys-Leu-Lys-Asp-Phe-Arg-Val-Tyr-Phe-Arg-Glu-Gly-
Arg-Asp-Gln-Leu-Trp-Lys-Gly-Pro-Gly-Glu-Leu-Leu-Trp-
Lys-Gly-Glu-Gly-Ala-Val-Leu-Val-Lys-Val-Gly-Thr-Asp
Ile-Lys-Y-Z
(XI) ROD 124C5X 
W-X-Lys-Leu-Lys-Asp-Phe-Arg-Val-Tyr-Phe-Arg-Glu-Gly-
Arg-Asp-Gln-Leu-Trp-Lys-Gly-Pro-Gly-Glu-Leu-Leu-Trp-
Lys-Gly-Glu-Gly-Als-Val-Leu-Val-Lys-Val-Gly-Y-Z
 wherein W is either a H of the amino terminal NH₂ group of the polypeptide or one or more additional amino acids bonded to the amino terminal NH₂ group of the polypeptide, the additional amino acids being selected to facilitate coupling of the polypeptide to a carrier protein or to a support; X is absent, Cys-Gly-Gly; or Lys-Lys, Y is absent, Cys- or one or more amino acids added to facilitate coupling; and Z is OH or NH;
(b) determining whether immunospecific binding has occurred between the polypeptide and an antibody component of the body fluid in which the detection of the immune complex indicates the presence of antibodies to HIV in the body fluid.

9. A polypeptide composition, immunoreactive to antibodies to HIV, comprising at least one of the following amino acid sequences:
(V) ROD 124 E1 
W-X-Lys-Leu-Lys-Asp-Phe-Arg-Val-Tyr-Phe-Arg-Glu-Gly-
Arg-Asp-Gln-Leu-Trp-Lys-Gly-Pro-Gly-Glu-Leu-Leu-Trp-
Lys-Gly-Glu-Gly-Ala-Y-Z
(VII) ROD 124C2X 
W-X-Lys-Leu-Lys-Asp-Phe-Arg-Val-Tyr-Phe-Arg-Glu-Gly-
Arg-Asp-Gln-Leu-Trp-Lys-Gly-Pro-Gly-Glu-Leu-Leu-Trp-
Lys-Gly-Glu-Gly-Ala-Val-Leu-Val-Lys-Val-Gly-Thr-Asp-
Ile-Lys-Y-Z
(VIII) ROD C1X 
W-X-Tyr-Phe-Arg-Glu-Gly-Arg-Asp-Gln-Leu-Trp-Lys-Gly-
Pro-Gly-Glu-Leu-Leu-Trp-Lys-Gly-Glu-Gly-Ala-Val-Leu-
Val-Lys-Val-Gly-Thr-Asp-Ile-Lys-Y-Z
(IX) ROD C3X 
X-Lys-Leu-Lys-Asp-Phe-Arg-Val-Tyr-Phe-Arg-Glu-Gly-
Arg-Asp-Gln-Leu-Trp-Lys-Gly-Pro-Gly-Glu-Leu-Leu-Trp-
Lys-Gly-Glu-Gly-Ala-Val-Leu-Val-Lys-Val-Gly-Thr-Asp
Ile-Lys-Y-Z
(XI) ROD 124C5X 
W-X-Lys-Leu-Lys-Asp-Phe-Arg-Val-Tyr-Phe-Arg-Glu-Gly-
Arg-Asp-Gln-Leu-Trp-Lys-Gly-Pro-Gly-Glu-Leu-Leu-Trp-
Lys-Gly-Glu-Gly-Als-Val-Leu-Val-Lys-Val-Gly-Y-Z
wherein W is either a H of the amino terminal NH₂ group of the polypeptide or one or more additional amino acids bonded to the amino terminal NH₂ group of the polypeptide, the additional amino acids being selected to facilitate coupling of the polypeptide to a carrier protein or to a support; X is absent, Cys-Gly-Gly; or Lys-Lys, Y is absent, Cys- or one or more amino acids added to facilitate coupling; and Z is OH or NH₂.

10. The polypeptide composition according to claim 9, wherein said polypeptide has formula (V) ROD 124 E1.

11. The polypeptide composition according to claim 9, wherein said polypeptide has formula (VII) ROD 124C2X.

12. The polypeptide composition according to claim 9, wherein said polypeptide has formula (VIII) ROD C1X.

13. The polypeptide composition of claim 9, wherein said polypeptide has formula (IX) ROD C3X.

14. The polypeptide composition of claim 9, wherein said polypeptide has formula (XI) ROD 124C5X.

15. A test kit for determining the presence of antibodies to HIV in a body fluid, comprising an antigen and ancillary reagents suitable for use in detecting the presence of antibodies to the antigen in said fluid; wherein said antigen is the polypeptide of claim 9.

## Patentansprüche

1. Verfahren zum Bestimmen des Vorhandenseins von HIV-Antikörpern in einer Körperflüssigkeit, umfassend:
(a) unter Bedingungen, die immunspezifische Bindung zulassen, zum Herstellen einer Reaktionsmischung, in Kontakt bringen der Körperflüssigkeit mit einer Zusammensetzung, die mindestens ein Polypeptid beinhaltet, das aus der Gruppe mit den folgenden Aminosäuresequenzen gewählt ist:
(V) ROD 124 E1 
W-X-Lys-Leu-Lys-Asp-Phe-Arg-Val-Tyr-Phe-Arg-Glu-Gly-Arg-Asp-Gln-Leu-Trp-Lys-
Gly-Pro-Gly-Glu-Leu-Leu-Trp-Lys-Gly-Glu-Gly-Ala-Y-Z
(VII) ROD 124C2X 
W-X-Lys-Leu-Lys-Asp-Phe-Arg-Val-Tyr-Phe-Arg-Glu-Gly-Arg-Asp-Gln-Leu-Trp-Lys-
Gly-Pro-Gly-Glu-Leu-Leu-Trp-Lys-Gly-Glu-Gly-Ala-Val-Leu-Val-Lys-Val-Gly-Thr-Asp-
Ile-Lys-Y-Z
(VIII) ROD C1X 
W-X-Tyr-Phe-Arg-Glu-Gly-Arg-Asp-Gln-Leu-Trp-Lys-Gly-Pro-Gly-Glu-Leu-Leu-Trp-
Lys-Gly-Glu-Gly-Ala-Val-Leu-Val-Lys-Val-Gly-Thr-Asp-Ile-Lys-Y-Z
(IX) ROD C3X 
X-Lys-Leu-Lys-Asp-Phe-Arg-Val-Tyr-Phe-Arg-Glu-Gly-Arg-Asp-Gln-Leu-Trp-Lys-Gly-
Pro-Gly-Glu-Leu-Leu-Trp-Lys-Gly-Glu-Gly-Ala-Val-Leu-Val-Lys-Val-Gly-Thr-Asp I l e-
Lys-Y-Z
(XI) ROD 124 C5X 
W-X-Lys-Leu-Lys-Asp-Phe-Arg-Val-Tyr-Phe-Arg-Glu-Gly-Arg-Asp-Gln-Leu-Trp-Lys-
Gly-Pro-Gly-Glu-Leu-Leu-Trp-Lys-Gly-Glu-Gly-Als-Val-Leu-Val-Lys-Val-Gly-Y-Z
wobei W entweder ein H der NH₂-Gruppe am Aminoende des Polypeptids oder eine oder mehrere zusätzliche Aminosäure(n) ist, das/die an die NH₂-Gruppe am Aminoende des Polypeptids gebunden ist/sind, wobei die zusätzlichen Aminosäuren so ausgewählt werden,
dass die Bindung des Polypeptids an ein Trägerprotein oder an eine Trägersubstanz erleichtert wird; X abwesend ist, Cys-Gly-Gly; oder Lys-Lys, Y abwesend ist, Cys- oder eine oder mehrere Aminosäure(n) hinzugefügt wird/werden, um die Bindung zu erleichtern; und Z entweder OH oder NH ist;
(b) Erkennen, ob eine immunspezifische Bindung stattgefunden hat zwischen dem Polypeptid und einer Antikörperkomponente der Körperflüssigkeit, in der ein Immunkomplex gebildet wird und in der die Erkennung des Immunkomplexes auf das Vorhandensein von HIV-Antikörpern in der Körperflüssigkeit hinweist.

2. Verfahren nach Anspruch 1, bei dem das Polypeptid zu einem Träger-Makromolekül konjugiert wird.

3. Verfahren nach Anspruch 1, bei dem das Polypeptid immobilisiert wird.

4. Verfahren nach Anspruch 1, bei dem die immunspezifische Bindung durch Immunpräzipitation erkannt wird.

5. Verfahren nach Anspruch 1, bei dem die Zusammensetzung mindestens ein Polypeptid, das aus einem Polymerase-Protein von HIV-2 gewählt wurde, und eines, das aus einem Polymerase-Protein von HIV-1 gewählt wurde, beinhaltet.

6. Verfahren nach Anspruch 1, bei dem die immunspezifische Bindung zwischen dem Polypeptid oder Protein und der Antikörperkomponente der Körperflüssigkeit erkannt wird durch:
(i) Entfernen nicht gebundener Komponenten aus den Immunkomplexen, die in der Immunreaktionsmischung gebildet wurden;
(ii) Hinzufügen eines markierten Antikörpers zu der Immunreaktionsmischung, wobei der markierte Antikörper immunspezifisch an eine Komponente des Immunkomplexes binden kann und die Markierung ein erkennbares Signal bereitstellt; und
(iii) Bestimmen, ob der markierte Antikörper an die Immunkomplexe bindet.

7. Verfahren nach Anspruch 6, bei dem die Markierung ein Enzym, das durch Hinzufügen des Enzymsubstrats erkannt wird, eine Radiomarkierung oder eine fluoreszierende Markierung umfasst.

8. Verfahren zum Bestimmen des Vorhandenseins von HIV-2-Antikörpern in einer Körperflüssigkeit, umfassend:
(a) unter Bedingungen, die immunspezifische Bindung zulassen, zum Herstellen einer Reaktionsmischung, in Kontakt bringen der Körperflüssigkeit mit einer Zusammensetzung, die mindestens ein Polypeptid beinhaltet, das aus der Gruppe mit den folgenden Aminosäuresequenzen gewählt ist:
(V) ROD 124 E1 
W-X-Lys-Leu-Lys-Asp-Phe-Arg-Val-Tyr-Phe-Arg-Glu-Gly-Arg-Asp-Gln-Leu-Trp-Lys-
Gly-Pro-Gly-Glu-Leu-Leu-Trp-Lys-Gly-Glu-Gly-Ala-Y-Z
(VII) ROD 124 C2X 
W-X-Lys-Leu-Lys-Asp-Phe-Arg-Val-Tyr-Phe-Arg-Glu-Gly-Arg-Asp-Gln-Leu-Trp-Lys-
Gly-Pro-Gly-Glu-Leu-Leu-Trp-Lys-Gly-Glu-Gly-Ala-Val-Leu-Val-Lys-Val-Gly-Thr-Asp-
Ile-Lys-Y-Z
(VIII) ROD C1X 
W-X-Tyr-Phe-Arg-Glu-Gly-Arg-Asp-Gln-Leu-Trp-Lys-Gly-Pro-Gly-Glu-Leu-Leu-Trp-
Lys-Gly-Glu-Gly-Ala-Val-Leu-Val-Lys-Val-Gly-Thr-Asp-Ile-Lys-Y-Z
(IX) ROD C3X 
X-Lys-Leu-Lys-Asp-Phe-Arg-Val-Tyr-Phe-Arg-Glu-Gly-Arg-Asp-Gln-Leu-Trp-Lys-Gly-
Pro-Gly-Glu-Leu-Leu-Trp-Lys-Gly-Glu-Gly-Ala-Val-Leu-Val-Lys-Val-Gly-Thr-Asp Ile-
Lys-Y-Z
(XI) ROD 124C5X 
W-X-Lys-Leu-Lys-Asp-Phe-Arg-Val-Tyr-Phe-Arg-Glu-Gly-Arg-Asp-Gln-Leu-Trp-Lys-
Gly-Pro-Gly-Glu-Leu-Leu-Trp-Lys-Gly-Glu-Gly-Als-Val-Leu-Val-Lys-Val-Gly-Y-Z
wobei W entweder ein H der NH₂-Gruppe am Aminoende des Polypeptids oder eine oder mehrere zusätzliche Aminosäure(n) ist, das/die an die NH₂-Gruppe am Aminoende des Polypeptids gebunden ist/sind, wobei die zusätzlichen Aminosäuren so ausgewählt werden, dass die Bindung des Polypeptids an ein Trägerprotein oder an eine Trägersubstanz erleichtert wird; X abwesend ist, Cys-Gly-Gly; oder Lys-Lys, Y abwesend ist, Cys- oder eine oder mehrere Aminosäure(n) hinzugefügt wird/werden, um die Bindung zu erleichtern; und Z entweder OH oder NH ist;
(b) Erkennen, ob eine immunspezifische Bindung stattgefunden hat zwischen dem Polypeptid und einer Antikörperkomponente der Körperflüssigkeit, in der die Erkennung des Immunkomplexes auf das Vorhandensein von HIV-Antikörpern in der Körperflüssigkeit hinweist.

9. Auf HIV-Antikörper immunreaktive Polypeptid-Zusammensetzung, die mindestens eine der folgenden Aminosäuresequenzen beinhaltet:
(V) ROD 124 E1 
W-X-Lys-Leu-Lys-Asp-Phe-Arg-Val-Tyr-Phe-Arg-Glu-Gly-Arg-Asp-Gln-Leu-Trp-Lys-
Gly-Pro-Gly-Glu-Leu-Leu-Trp-Lys-Gly-Glu-Gly-Ala-Y-Z
(VII) ROD 124 C2X 
W-X-Lys-Leu-Lys-Asp-Phe-Arg-Val-Tyr-Phe-Arg-Glu-Gly-Arg-Asp-Gln-Leu-Trp-Lys-
Gly-Pro-Gly-Glu-Leu-Leu-Trp-Lys-Gly-Glu-Gly-Ala-Val-Leu-Val-Lys-Val-Gly-Thr-Asp-
Ile-Lys-Y-Z
(VIII) ROD C1X 
W-X-Tyr-Phe-Arg-Glu-Gly-Arg-Asp-Gln-Leu-Trp-Lys-Gly-Pro-Gly-Glu-Leu-Leu-Trp-
Lys-Gly-Glu-Gly-Ala-Val-Leu-Val-Lys-Val-Gly-Thr-Asp-Ile-Lys-Y-Z
(IX) ROD C3X 
X-Lys-Leu-Lys-Asp-Phe-Arg-Val-Tyr-Phe-Arg-Glu-Gly-Arg-Asp-Gln-Leu-Trp-Lys-Gly-
Pro-Gly-Glu-Leu-Leu-Trp-Lys-Gly-Glu-Gly-Ala-Val-Leu-Val-Lys-Val-Gly-Thr-Asp Ile-
Lys-Y-Z
(XI) ROD 124 C5X 
W-X-Lys-Leu-Lys-Asp-Phe-Arg-Val-Tyr-Phe-Arg-Glu-Gly-Arg-Asp-Gln-Leu-Trp-Lys-
Gly-Pro-Gly-Glu-Leu-Leu-Trp-Lys-Gly-Glu-Gly-Als-Val-Leu-Val-Lys-Val-Gly-Y-Z
wobei W entweder ein H der NH₂-Gruppe am Aminoende des Polypeptids oder eine oder mehrere zusätzliche Aminosäure(n) ist, das/die an die NH₂-Gruppe am Aminoende des Polypeptids gebunden ist/sind, wobei die zusätzlichen Aminosäuren so ausgewählt werden, dass die Bindung des Polypeptids an ein Trägerprotein oder an eine Trägersubstanz erleichtert wird; X abwesend ist, Cys-Gly-Gly; oder Lys-Lys, Y abwesend ist, Cys- oder eine oder mehrere Aminosäure(n) hinzugefügt wird/werden, um die Bindung zu erleichtern; und Z entweder OH oder NH₂ ist.

10. Polypeptid-Zusammensetzung nach Anspruch 9, wobei das Polypeptid die Formel (V) ROD 124 E1 hat.

11. Polypeptid-Zusammensetzung nach Anspruch 9, wobei das Polypeptid die Formel (VII) ROD 124C2X hat.

12. Polypeptid-Zusammensetzung nach Anspruch 9, wobei das Polypeptid die Formel (VIII) ROD C1X hat.

13. Polypeptid-Zusammensetzung nach Anspruch 9, wobei das Polypeptid die Formel (IX) ROD C3X hat.

14. Polypeptid-Zusammensetzung nach Anspruch 9, wobei das Polypeptid die Formel (XI) ROD 124C5X hat.

15. Test-Kit zum Bestimmen des Vorhandenseins von HIV-Antikörpern in einer Körperflüssigkeit, umfassend ein Antigen und zusätzliche Reagenzien, die geeignet sind, das Vorhandensein von Antikörpern zu dem Antigen in der betreffenden Flüssigkeit zu erkennen, wobei das Antigen das Polypeptid von Anspruch 9 ist.

## Revendications

1. Procédé permettant de déterminer la présence d'anticorps anti-VIH dans un fluide corporel, comprenant les étapes suivantes :
(a) mise en contact, dans des conditions permettant à une liaison immunospécifique de former un mélange réactif, du fluide corporel avec une composition contenant au moins un polypeptide sélectionné parmi le groupe composé des séquences amino acides suivantes :
(V) ROD 124 E1 
W-X-Lys-Leu-Lys-Asp-Phe-Arg-Val-Tyr-Phe-Arg-Glu-Gly-
Arg-Asp-Gln-Leu-Trp-Lys-Gly-Pro-Gly-Glu-Leu-Leu-Trp-
Lys-Gly-Glu-Gly-Ala-Y-Z
(VII) ROD 124C2X 
W-X-Lys-Leu-Lys-Asp-Phe-Arg-Val-Tyr-Phe-Arg-Glu-Gly-
Arg-Asp-Gln-Leu-Trp-Lys-Gly-Pro-Gly-Glu-Leu-Leu-Trp-
Lys-Gly-Glu-Gly-Ala-Val-Leu-Val-Lys-Val-Gly-Thr-Asp-
Ile-Lys-Y-Z
(VII) ROD CIX 
W-X-Tyr-Phe-Arg-Glu-Gly-Arg-Asp-Gln-Leu-Trp-Lys-Gly-
Pro-Gly-Glu-Leu-Leu-Trp-Lys-Gly-Glu-Gly-Ala-Val-Leu-
Val-Lys-Val-Gly-Thr-Asp-Ile-Lys-Y-Z
(IX) ROD C3X 
X-Lys-Leu-Lys-Asp-Phe-Arg-Val-Tyr-Phe-Arg-Glu-Gly-
Arg-Asp-Gln-Leu-Trp-Lys-Gly-Pro-Gly-Glu-Leu-Leu-Trp-
Lys-Gly-Glu-Gly-Ala-Val-Leu-Val-Lys-Val-Gly-Thr-Asp
Ile-Lys-Y-Z
(XI) ROD 124C5X 
W-X-Lys-Leu-Lys-Asp-Phe-Arg-Val-Tyr-Phe-Arg-Glu-Gly-
Arg-Asp-Gln-Leu-Trp-Lys-Gly-Pro-Gly-Glu-Leu-Leu-Trp-
Lys-Gly-Glu-Gly-Als-Val-Leu-Val-Lys-Val-Gly-Y-Z
dans lesquelles W est soit un H du groupe NH₂ amino-terminal du polypeptide ou un ou plusieurs amino acides supplémentaires liés au groupe NH₂ amino-terminal du polypeptide, les amino acides supplémentaires étant sélectionnés de sorte à faciliter le couplage du polypeptide avec une protéine porteuse ou un support ; X est absent, Cys-Gly-Gly ou Lys-Lys ; Y est absent, Cys- ou un ou plusieurs amino acides ajoutés pour faciliter le couplage ; et Z est OH ou NH ;
(b) détection de la formation ou non de la liaison immunospécifique entre le polypeptide et un composant d'anticorps du fluide corporel, dans laquelle un complexe immun est formé et la détection du complexe immun indique la présence d'anticorps anti-VIH dans le fluide corporel.

2. Procédé selon la revendication 1, dans lequel le polypeptide est conjugué à une macromolécule porteuse.

3. Procédé selon la revendication 1, dans lequel le polypeptide est immobilisé.

4. Procédé selon la revendication 1, dans lequel la liaison immunospécifique est détectée par immunoprécipitation.

5. Procédé selon la revendication 1, dans lequel la composition comprend au moins un polypeptide sélectionné parmi une protéine polymérase de VIH-2 et un polypeptide sélectionné parmi une protéine polymérase de VIH-1.

6. Procédé selon la revendication 1, dans lequel la liaison immunospécifique entre le polypeptide ou la protéine et le composant d'anticorps du fluide corporel est détectée en : (i) éliminant le composant non lié des complexes immuns formés dans le mélange immunoréactif ;
(ii) ajoutant un anticorps marqué au mélange immunoréactif, l'anticorps marqué pouvant se lier de manière immunospécifique à un composant des complexes immuns, et le marqueur fournissant un signal détectable ; et
(iii) déterminant si l'anticorps marqué se lie ou non aux complexes immuns.

7. Procédé selon la revendication 6, dans lequel le marqueur comprend une enzyme qui est détectée par l'ajout d'un substrat enzymatique, d'un radio-marqueur ou d'un marqueur fluorescent.

8. Procédé permettant de déterminer la présence d'anticorps anti-VIH-2 dans un fluide corporel, comprenant les étapes suivantes :
(a) mise en contact, dans des conditions permettant à une liaison immunospécifique de former un mélange réactif, du fluide corporel avec une composition contenant au moins un polypeptide sélectionné parmi le groupe composé des séquences amino acides suivantes :
(V) ROD 124 E1 
W-X-Lys-Leu-Lys-Asp-Phe-Arg-Val-Tyr-Phe-Arg-Glu-Gly-
Arg-Asp-Gln-Leu-Trp-Lys-Gly-Pro-Gly-Glu-Leu-Leu-Trp-
Lys-Gly-Glu-Gly-Ala-Y-Z
(VII) ROD 124C2X 
W-X-Lys-Leu-Lys-Asp-Phe-Arg-Val-Tyr-Phe-Arg-Glu-Gly-
Arg-Asp-Gln-Leu-Trp-Lys-Gly-Pro-Gly-Glu-Leu-Leu-Trp-
Lys-Gly-Glu-Gly-Ala-Val-Leu-Val-Lys-Val-Gly-Thr-Asp-
Ile-Lys-Y-Z
(VIII) ROD CIX 
W-X-Tyr-Phe-Arg-Glu-Gly-Arg-Asp-Gln-Leu-Trp-Lys-Gly-
Pro-Gly-Glu-Leu-Leu-Trp-Lys-Gly-Glu-Gly-Ala-Val-Leu-
Val-Lys-Val-Gly-Thr-Asp-Ile-Lys-Y-Z
(IX) ROD C3X 
X-Lys-Leu-Lys-Asp-Phe-Arg-Val-Tyr-Phe-Arg-Glu-Gly-
Arg-Asp-Gln-Leu-Trp-Lys-Gly-Pro-Gly-Glu-Leu-Leu-Trp-
Lys-Gly-Glu-Gly-Ala-Val-Leu-Val-Lys-Val-Gly-Thr-Asp
Ile-Lys-Y-Z
(XI) ROD 124C5X 
W-X-Lys-Leu-Lys-Asp-Phe-Arg-Val-Tyr-Phe-Arg-Glu-Gly-
Arg-Asp-Gln-Leu-Trp-Lys-Gly-Pro-Gly-Glu-Leu-Leu-Trp-
Lys-Gly-Glu-Gly-Als-Val-Leu-Val-Lys-Val-Gly-Y-Z
dans lesquelles W est soit un H du groupe NH₂ amino-terminal du polypeptide ou un ou plusieurs amino acides supplémentaires liés au groupe NH₂ amino-terminal du polypeptide, les amino acides supplémentaires étant sélectionnés de sorte à faciliter le couplage du polypeptide avec une protéine porteuse ou un support ; X est absent, Cys-Gly-Gly ou Lys-Lys ; Y est absent, Cys- ou un ou plusieurs amino acides ajoutés pour faciliter le couplage ; et Z est OH ou NH ;
(b) détermination de la formation ou non de la liaison immunospécifique entre le polypeptide et un composant d'anticorps du fluide corporel, dans laquelle la détection du complexe immun indique la présence d'anticorps anti-VIH dans le fluide corporel.

9. Composition de polypeptide, immunoréactive aux anticorps anti-VIH, comprenant au moins une des séquences amino acides suivantes :
(V) ROD 124 E1 
W-X-Lys-Leu-Lys-Asp-Phe-Arg-Val-Tyr-Phe-Arg-Glu-Gly-
Arg-Asp-Gln-Leu-Trp-Lys-Gly-Pro-Gly-Glu-Leu-Leu-Trp-
Lys-Gly-Glu-Gly-Ala-Y-Z
(VII) ROD 124C2X 
W-X-Lys-Leu-Lys-Asp-Phe-Arg-Val-Tyr-Phe-Arg-Glu-Gly-
Arg-Asp-Gln-Leu-Trp-Lys-Gly-Pro-Gly-Glu-Leu-Leu-Trp-
Lys-Gly-Glu-Gly-Ala-Val-Leu-Val-Lys-Val-Gly-Thr-Asp-
Ile-Lys-Y-Z
(VIII) ROD CIX 
W-X-Tyr-Phe-Arg-Glu-Gly-Arg-Asp-Gln-Leu-Trp-Lys-Gly-
Pro-Gly-Glu-Leu-Leu-Trp-Lys-Gly-Glu-Gly-Ala-Val-Leu-
Val-Lys-Val-Gly-Thr-Asp-Ile-Lys-Y-Z
(IX) ROD C3X 
X-Lys-Leu-Lys-Asp-Phe-Arg-Val-Tyr-Phe-Arg-Glu-Gly-
Arg-Asp-Gln-Leu-Trp-Lys-Gly-Pro-Gly-Glu-Leu-Leu-Trp-
Lys-Gly-Glu-Gly-Ala-Val-Leu-Val-Lys-Val-Gly-Thr-Asp
Ile-Lys-Y-Z
(XI) ROD 124C5X 
W-X-Lys-Leu-Lys-Asp-Phe-Arg-Val-Tyr-Phe-Arg-Glu-Gly-
Arg-Asp-Gln-Leu-Trp-Lys-Gly-Pro-Gly-Glu-Leu-Leu-Trp-
Lys-Gly-Glu-Gly-Als-Val-Leu- Val-Lys-Val-Gly-Y-Z
dans lesquelles W est soit un H du groupe NH₂ amino-terminal du polypeptide ou un ou plusieurs amino acides supplémentaires liés au groupe NH₂ amino-terminal du polypeptide, les amino acides supplémentaires étant sélectionnés de sorte à faciliter le couplage du polypeptide avec une protéine porteuse ou un support ; X est absent, Cys-Gly-Gly ou Lys-Lys ; Y est absent, Cys- ou un ou plusieurs amino acides ajoutés pour faciliter le couplage ; et Z est OH ou NH₂.

10. Composition de polypeptide selon la revendication 9, dans laquelle ledit polypeptide est de formule (V) ROD 124 E1.

11. Composition de polypeptide selon la revendication 9, dans laquelle ledit polypeptide est de formule (VII) ROD 124C2X.

12. Composition de polypeptide selon la revendication 9, dans laquelle ledit polypeptide est de formule (VIII) ROD C1X.

13. Composition de polypeptide selon la revendication 9, dans laquelle ledit polypeptide est de formule (IX) ROD C3X.

14. Composition de polypeptide selon la revendication 9, dans laquelle ledit polypeptide est de formule (XI) ROD 124C5X.

15. Kit d'essai permettant de déterminer la présence d'anticorps anti-VIH dans un fluide corporel, comprenant un antigène et des réactifs auxiliaires à utiliser pour détecter la présence d'anticorps anti-antigène dans ledit fluide, dans lequel ledit antigène est le polypeptide de la revendication 9.
